# EUROPEAN PATENT APPLICATION

(11) **EP 3 238 733 A1**
(43) Date of publication of application: **01.11.2017**
(21) Application number: 15863151.5
(22) Date of filing: 28.05.2015
(51) Int. Cl.: A61K 38/00, A61K 39/395, A61K 47/42, A61K 47/50, A61P 5/16, A61P 9/00, A61P 19/02, A61P 21/00, A61P 21/04, A61P 25/00, A61P 25/02, A61P 29/00, A61P 31/22, A61P 37/02, A61P 37/06, A61P 43/00, C07K 14/475, C07K 19/00

(54) **B CELL ACTIVATION INHIBITOR, AND THERAPEUTIC AGENT FOR AUTOIMMUNE DISEASES**

(30) Priority: 28.11.2014 JP 2014241193
(71) Applicant: The University of Tokyo, Tokyo 113-8654 (JP); Chugai Seiyaku Kabushiki Kaisha, Kita-ku Tokyo 115-8543 (JP)
(72) Inventor: FUJIO, Keishi, Tokyo 113-8654 (JP); OKAMURA, Tomohisa, Tokyo 113-8654 (JP); YAMAMOTO, Kazuhiko, Tokyo 113-8654 (JP); MORITA, Kaoru, Tokyo 113-8654 (JP); KOMAI, Toshihiko, Tokyo 113-8654 (JP); BAN, Nobuhiro, Kamakura-shi Kanagawa 247-8530 (JP); MATSUURA, Tetsu, Kamakura-shi Kanagawa 247-8530 (JP); NONAKA, Tatsuya, Kamakura-shi Kanagawa 247-8530 (JP)
(74) Representative: Vossius & Partner Patentanwälte Rechtsanwälte mbB
(86) International application number: PCT/JP2015/065466
(87) International publication number: WO 2016/084412

(57) **Abstract**

The inventors found that B cell activation is suppressed by TGF-β3 produced by LAG3⁺ Treg. They also discovered a B cell activation suppressor which contains TGF-β3 or a molecule having a TGF-β3 function, and a therapeutic agent for autoimmune diseases.

## Description

### Technical Field

The present invention relates to suppressors of B cell activation, therapeutic agents for autoimmune diseases, and medical kits comprising the therapeutic agents. More specifically, the present invention relates to suppressors of B cell activation comprising TGF-beta 3, and therapeutic agents for autoimmune diseases that use the suppressors. Furthermore, the present invention relates to therapeutic agents for autoimmune diseases that comprise a molecule formed by linking TGF-beta 3 with an antibody or an antibody fragment. The present invention also relates to medical kits comprising the above-mentioned therapeutic agents.

### Background Art

Autoantibodies are known to induce various autoimmune diseases, including systemic lupus erythematosus (SLE), which is characterized by severe inflammation in multiple organ systems (Non-Patent Document 1). The high-affinity autoantibodies primarily originate from self-reactive B cells that underwent somatic hypermutation in germinal centers (GCs) (Non-Patent Document 2). Follicular helper T (T_{FH}) cells expressing CXCR5 are helper T cells functionally specialized to provide help to B cells allowing the formation of GCs and the subsequent long-lived plasma cell differentiation. Therefore, regulation of the quality and quantity of T_{FH} cells and memory B cell populations in GCs is closely associated with pathology of autoimmune diseases. CD4⁺CD25⁺ regulatory T cells (CD25⁺ Treg) that express Foxp3 play key roles in the maintenance of self-tolerance and suppress the activation of conventional T cells and dendritic cells (Non-Patent Document 3). Moreover, several reports suggest the essential role of CD25⁺ Treg, including CD4⁺CD25⁺CXCR5⁺ follicular Treg (T_{FR}) (Non-Patent Document 2) and CD4⁺CD25⁺CD69⁻ Treg (Non-Patent Document 4), in the regulation of humoral immunity. These observations clearly indicate that CD25⁺ Treg systemically suppress autoimmunity; however, the pathology of diseases induced by the absence of functional CD25⁺ Treg is quite different from SLE (Non-Patent Documents 1 and 5) and the role of CD25⁺ Treg in SLE has not been clarified (Non-Patent Document 6). Recent advances in the studies of CD8⁺ Treg in mice have underscored the importance of Qa-1-restricted CD8⁺ Treg for the maintenance of B cell tolerance. Mice with functional impairment in CD8⁺ Treg exhibit a lupus-like disease pathology with a significant increase in T_{FH} (Non-Patent Document 7). The development of systemic autoimmune reactions in B6.*Yaa* mutant mice is associated with a pronounced defect in CD8⁺ Treg activity (Non-Patent Document 8). Nevertheless, the actual contribution of CD8⁺ Treg to the regulation of human autoimmune reactions remains unclear.

Early growth response gene 2 (Egr2), a zinc-finger transcription factor, plays a critical role in hindbrain development and myelination of the peripheral nervous system (Non-Patent Document 9). In T cells, Egr2 is important for the maintenance of T cell anergy by negatively regulating T cell activation (Non-Patent Document 10). The involvement of Egr2 in the control of systemic autoimmune reactions was first suggested by the observation that lymphocyte-specific Egr2-deficient mice develop a lupus-like disease with no impact on the development of Foxp3-expressing CD25⁺ Treg (Non-Patent Document 11). Moreover, mice deficient for both Egr2 and Egr3 in B and T cells present a lethal and early-onset systemic autoimmune disease, suggesting a synergistic role for Egr2 and Egr3 in controlling B cell tolerance (Non-Patent Document 12). The present inventors and their collaborators have shown that polymorphisms in *EGR2* influence SLE susceptibility in humans (Non-Patent Document 13). The present inventors have previously identified the existence of Egr2-controlled CD4⁺CD25⁻LAG3⁺ Treg (LAG3⁺ Treg) (Non-Patent Document 14). LAG3 is a CD4-related molecule that binds to MHC class II, and the binding induces ITAM-mediated inhibitory signaling in dendritic cells (Non-Patent Document 15). Approximately 2% of the CD4⁺CD25⁻T cell population in the spleen expresses LAG3. These LAG3⁺ Treg produce high levels of interleukin (IL)-10 and are suppressive in a murine model of colitis in an IL-10-dependent manner. Unlike CD25⁺ Treg, high affinity interactions with selecting peptide/MHC ligands expressed in the thymus do not induce the development of LAG3⁺ Treg. Recently, Gagliani *et al*. reported that concomitant expression of LAG3 and CD49b is specific for IL-10-producing type 1 T regulatory (Tr1) cells (Non-Patent Document 16), suggesting that LAG3 is one of the markers of IL-10-producing Foxp3-independent CD4⁺ Treg.

### Prior Art Documents

### [Non-Patent Documents]

[Non-Patent Document 1] Tsokos, G.C. Systemic lupus erythematosus. N. Engl. J. Med. 365, 2110-2121 (2011).
[Non-Patent Document 2] Linterman, M.A., et al. Foxp3+ follicular regulatory T cells control the germinal center response. Nat. Med. 17, 975-982 (2011).
[Non-Patent Document 3] Sakaguchi, S. & Powrie, F. Emerging challenges in regulatory T cell function and biology. Science 317, 627-629 (2007).
[Non-Patent Document 4] Lim, H.W., Hillsamer, P., Banham, A.H. & Kim, C.H. Cutting edge: direct suppression of B cells by CD4+ CD25+ regulatory T cells. J. Immunol. 175, 4180-4183 (2005).
[Non-Patent Document 5] Okamura, T., Fujio, K., Sumitomo, S. & Yamamoto, K. Roles of LAG3 and EGR2 in regulatory T cells. Ann. Rheum. Dis. 71 Suppl 2, i96-100 (2012).
[Non-Patent Document 6] Suen, J.L. & Chiang, B.L. CD4(+)FoxP3(+) regulatory T-cells in human systemic lupus erythematosus. J. Formos. Med. Assoc. 111, 465-470 (2012).
[Non-Patent Document 7] Kim, H.J., Verbinnen, B., Tang, X., Lu, L. & Cantor, H. Inhibition of follicular T-helper cells by CD8(+) regulatory T cells is essential for self tolerance. Nature 467. 328-332 (2010).
[Non-Patent Document 8] Kim, H.J., et al. CD8+ T regulatory cells express the Ly49 Class I MHC receptor and are defective in autoimmune prone B6-Yaa mice. Proc. Natl. Acad. Sci. USA 108, 2010-2015 (2011).
[Non-Patent Document 9] Gillian, A.L. & Svaren, J. The Ddx20/DP103 dead box protein represses transcriptional activation by Egr2/Krox-20. J. Biol. Chem. 279, 9056-9063 (2004).
[Non-Patent Document 10] Safford, M., et al. Egr-2 and Egr-3 are negative regulators of T cell activation. Nat. Immunol. 6,472-480 (2005).
[Non-Patent Document 11] Zhu, B., et al. Early growth response gene 2 (Egr-2) controls the self-tolerance of T cells and prevents the development of lupuslike autoimmune disease. J. Exp. Med. 205,2295-2307 (2008).
[Non-Patent Document 12] Li, S., et al. The transcription factors Egr2 and Egr3 are essential for the control of inflammation and antigen-induced proliferation of B and T cells. Immunity 37, 685-696 (2012).
[Non-Patent Document 13] Myouzen, K., et al. Regulatory polymorphisms in EGR2 are associated with susceptibility to systemic lupus erythematosus. Hum. Mol. Genet. 19, 2313-2320 (2010).
[Non-Patent Document 14] Okamura, T., et al. CD4+CD25-LAG3+ regulatory T cells controlled by the transcription factor Egr-2. Proc. Natl. Acad. Sci. U S A 106, 13974-13979 (2009).
[Non-Patent Document 15] Liang, B., et al. Regulatory T cells inhibit dendritic cells by lymphocyte activation gene-3 engagement of MHC class II. J. Immunol. 180, 5916-5926 (2008).
[Non-Patent Document 16] Gagliani, N., et al. Coexpression of CD49b and LAG-3 identifies human and mouse T regulatory type 1 cells. Nat. Med. 19, 739-746 (2013).
[Non-Patent Document 17] Cohen, P.L. & Eisenberg, R.A. Lpr and gld: single gene models of systemic autoimmunity and lymphoproliferative disease. Annu. Rev. Immunol. 9, 243-269 (1991).
[Non-Patent Document 18] Hasegawa, H., et al. Therapy for pneumonitis and sialadenitis by accumulation of CCR2-expressing CD4+CD25+ regulatory T cells in MRL/lpr mice. Arthritis Res. Ther. 9, R15 (2007).
[Non-Patent Document 19] Iwasaki, Y., et al. Egr-2 transcription factor is required for Blimp-1-mediated IL-10 production in IL-27-stimulated CD4+ T cells. Eur. J. Immunol. 43, 1063-1073 (2013).
[Non-Patent Document 20] Spender, L.C., et al. TGF-beta induces apoptosis in human B cells by transcriptional regulation of BIK and BCL-XL. Cell Death Differ. 16, 593-602 (2009).
[Non-Patent Document 21] Nelms, K., Keegan, A.D., Zamorano, J., Ryan, J.J. & Paul, W.E. The IL-4 receptor: signaling mechanisms and biologic functions. Annu. Rev. Immunol. 17, 701-738 (1999).
[Non-Patent Document 22] Kurosaki, T. Genetic analysis of B cell antigen receptor signaling. Annu. Rev. Immunol. 17, 555-592 (1999).
[Non-Patent Document 23] Zhang, W., et al. Aberrant CD40-induced NF-kappaB activation in human lupus B lymphocytes. PLoS One 7, e41644 (2012).
[Non-Patent Document 24] Lee, Y., et al. Induction and molecular signature of pathogenic TH17 cells. Nat. Immunol. 13,991-999 (2012).
[Non-Patent Document 25] Keir, M.E., Butte, M.J., Freeman, G.J. & Sharpe, A.H. PD-1 and its ligands in tolerance and immunity. Annu. Rev. Immunol. 26, 677-704 (2008).
[Non-Patent Document 26] Haas, K.M. Programmed cell death 1 suppresses B-1b cell expansion and long-lived IgG production in response to T cell-independent type 2 antigens. J. Immunol. 187, 5183-5195 (2011).
[Non-Patent Document 27] Okazaki, T. & Honjo, T. The PD-1-PD-L pathway in immunological tolerance. Trends Immunol. 27, 195-201 (2006).
[Non-Patent Document 28] Okazaki, T., et al. PD-1 and LAG-3 inhibitory co-receptors act synergistically to prevent autoimmunity in mice. J. Exp. Med. 208, 395-407 (2011).
[Non-Patent Document 29] Woo, S.R., et al. Immune inhibitory molecules LAG-3 and PD-1 synergistically regulate T-cell function to promote tumoral immune escape. Cancer Res. 72, 917-927 (2012).
[Non-Patent Document 30] Ritz, O., et al. STAT6 activity is regulated by SOCS-1 and modulates BCL-XL expression in primary mediastinal B-cell lymphoma. Leukemia 22,2106-2110 (2008).
[Non-Patent Document 31] Good-Jacobson, K.L., et al. PD-1 regulates germinal center B cell survival and the formation and affinity of long-lived plasma cells. Nat. Immunol. 11, 535-542 (2010).
[Non-Patent Document 32] Stumhofer, J.S., et al. Interleukins 27 and 6 induce STAT3-mediated T cell production ofinterleukin 10. Nat. Immunol. 8, 1363-1371 (2007).
[Non-Patent Document 33] Miyara, M., et al. Functional delineation and differentiation dynamics of human CD4+ T cells expressing the FoxP3 transcription factor. Immunity 30, 899-911 (2009).
[Non-Patent Document 34] Chikuma, S., Suita, N., Okazaki, I.M., Shibayama, S. & Honjo, T. TRIM28 prevents autoinflammatory T cell development in vivo. Nat. Immunol. 13, 596-603 (2012).
[Non-Patent Document 35] Yoshimura, A., Wakabayashi, Y & Mori, T. Cellular and molecular basis for the regulation of inflammation by TGF-beta. J. Biochem. 147, 781-792 (2010).
[Non-Patent Document 36] Shull, M.M., et al. Targeted disruption of the mouse transforming growth factor-beta 1 gene results in multifocal inflammatory disease. Nature 359, 693-699 (1992).
[Non-Patent Document 37] Rubtsov, Y.P. & Rudensky, A.Y. TGFbeta signalling in control of T-cell-mediated self-reactivity. Nat. Rev. Immunol. 7, 443-453 (2007).
[Non-Patent Document 38] Kehrl, J.H., Thevenin, C., Rieckmann, P. & Fauci, A.S. Transforming growth factor-beta suppresses human B lymphocyte Ig production by inhibiting synthesis and the switch from the membrane form to the secreted form of Ig mRNA. J. Immunol. 146, 4016-4023 (1991).
[Non-Patent Document 39] Lomo, J., Blomhoff, H.K., Beiske, K., Stokke, T. & Smeland, E.B. TGF-beta 1 and cyclic AMP promote apoptosis in resting human B lymphocytes. J. Immunol. 154, 1634-1643 (1995).
[Non-Patent Document 40] Lohr, J., Knoechel, B. & Abbas, A.K. Regulatory T cells in the periphery. Immunol. Rev. 212, 149-162 (2006).
[Non-Patent Document 41] Chen, Y, Kuchroo, V.K., Inobe, J., Hafler, D.A. & Weiner, H.L. Regulatory T cell clones induced by oral tolerance: suppression of autoimmune encephalomyelitis. Science 265, 1237-1240 (1994).
[Non-Patent Document 42] Wing, J.B. & Sakaguchi, S. Foxp3+ Treg cells in humoral immunity. Int. Immunol. 26, 61-69 (2013).
[Non-Patent Document 43] Huber, S., et al. Th17 cells express interleukin-10 receptor and are controlled by Foxp3 and Foxp3+ regulatory CD4+ T cells in an interleukin-10-dependent manner. Immunity 34, 554-565 (2011).
[Non-Patent Document 44] Fuchs, A., Atkinson, J.P., Fremeaux-Bacchi, V. & Kemper, C. CD46-induced human Treg enhance B-cell responses. Eur. J. Immunol. 39, 3097-3109 (2009).
[Non-Patent Document 45] Sugiyama, N., et al. Amelioration of human lupus-like phenotypes in MRL/lpr mice by overexpression of interleukin 27 receptor alpha (WSX-1). Ann. Rheum. Dis. 67, 1461-1467 (2008).
[Non-Patent Document 46] Chavele, K.M. & Ehrenstein, M.R. Regulatory T-cells in systemic lupus erythematosus and rheumatoid arthritis. FEBS Lett. 585,3603-3610 (2011).
[Non-Patent Document 47] Nishimura, H., et al. Autoimmune dilated cardiomyopathy in PD-1 receptor-deficient mice. Science 291, 319-322 (2001).
[Non-Patent Document 48] Taillebourg, E., Buart, S. & Charnay, P. Conditional, floxed allele of the Krox20 gene. Genesis 32, 112-113 (2002).
[Non-Patent Document 49] Atkinson, C., Qiao, F., Song, H., Gilkeson, G.S. & Tomlinson, S. Low-dose targeted complement inhibition protects against renal disease and other manifestations of autoimmune disease in MRL/lpr mice. J. Immunol. 180, 1231-1238 (2008).
[Non-Patent Document 50] Nitta, Y., et al. Systemic delivery of interleukin 10 by intramuscular injection of expression plasmid DNA prevents autoimmune diabetes in nonobese diabetic mice. Hum. Gene Ther. 9, 1701-1707 (1998).
[Non-Patent Document 51] Hall, B., et al. Transforming growth factor-β3 (TGF-β3) knock-in ameliorates inflammation due to TGF-β1 deficiency while promoting glucose tolerance. J Biol Chem 2013;288 32074-32092
[Non-Patent Document 52] Lee, Y, et al. Induction and molecular signature of pathogenic TH17 cells.Nat Immunol. 2012;13:991-999
[Non-Patent Document 53] Kontermann, RE. Strategies for extended serum half-life of protein therapeutics. Current Oppinion in Biotechnology. 2011;6:868-876

### Summary of the Invention

### [Problems to be Solved by the Invention]

The relationship between Egr2 and systemic autoimmune reactions mediated by autoantibodies has suggested the involvement of Egr2-expressing LAG3⁺ Treg in B cell response regulation. However, the mechanism of humoral immunity regulation by LAG3⁺ Treg has not been elucidated, and in particular, the factors that mediate suppression of B cell activation by LAG3⁺ Treg have not been identified.

An objective of the present invention is to elucidate the mechanism of action by LAG3⁺ Treg, and also to provide novel suppressors of B cell activation and therapeutic agents for autoimmune diseases based on this finding.

### [Means for Solving the Problems]

As a result of dedicated research, the present inventors demonstrated that transforming growth factor-β3 (TGF-β3) produced by LAG3⁺ Treg is involved in the regulation of B-cell response. In addition, the present inventors confirmed that introduction of TGF-β3 significantly improves the pathology of MRL/*lpr* mice which are model mice for SLE pathology. Furthermore, the present inventors discovered that suppression of B cell activation through TGF-β3 may also take place in human LAG3⁺ Treg through the same mechanism as in mouse LAG3⁺ Treg.

The present invention is based on these novel findings, and provides suppressors and therapeutic agents that use TGF-β3.

More specifically, the present invention relates to the following:
[1] a suppressor of B cell activation, which comprises TGF-β3;
[2] the suppressor of [1], wherein the B cell is an autoreactive B cell;
[3] the suppressor of [1] or [2], wherein the TGF-β3 suppresses antibody production by B cells;
[4] a therapeutic agent for an autoimmune disease, which comprises the suppressor of any one of [1] to [3];
[5] a therapeutic agent for an autoimmune disease, which comprises a molecule in which TGF-β3 is linked with an antibody or an antibody fragment;
[6] the therapeutic agent of [5], wherein the antibody or the antibody fragment comprises a variable region that recognizes a B cell;
[7] the therapeutic agent of [6], wherein the variable region recognizes the B cell using any one or more of CD19, CD20, CD40, CD22, IL21R, BAFF-R, BCMA, TACI, CD27, and CD138 as a marker;
[8] a therapeutic agent for an autoimmune disease, which comprises a multispecific antibody comprising a first variable region that recognizes TGF-β3 and a second variable region that recognizes a B cell;
[9] the therapeutic agent of any one of [4] to [8], wherein the autoimmune disease is any one of systemic lupus erythematosus, pemphigus, multiple sclerosis, neuromyelitis optica, ANCA-associated vasculitis, rheumatoid arthritis, organ transplant rejection, Sjogren's syndrome, juvenile dermatomyositis, myasthenia gravis, or autoimmune thyroid disease including Graves' disease and Hashimoto's thyroiditis; and
[10] a medical kit comprising the therapeutic agent of any one of [4] to [8].

The present invention also relates to the following:
[11] a method for suppressing B cell activation, wherein the method comprises administering TGF-β3; a molecule in which TGF-β3 is linked with an antibody or an antibody fragment; or a multispecific antibody comprising a first variable region that recognizes TGF-β3 and a second variable region that recognizes a B cell;
[12] a method for treating an autoimmune disease, wherein the method comprises administering TGF-β3; a molecule in which TGF-β3 is linked with an antibody or an antibody fragment; or a multispecific antibody comprising a first variable region that recognizes TGF-β3 and a second variable region that recognizes a B cell;
[13] TGF-β3; a molecule in which TGF-β3 is linked with an antibody or an antibody fragment; or a multispecific antibody comprising a first variable region that recognizes TGF-β3 and a second variable region that recognizes a B cell for use in suppressing B cell activation;
[14] TGF-β3; a molecule in which TGF-β3 is linked with an antibody or an antibody fragment; or a multispecific antibody comprising a first variable region that recognizes TGF-β3 and a second variable region that recognizes a B cell for use in treating an autoimmune disease;
[15] use of TGF-β3; a molecule in which TGF-β3 is linked with an antibody or an antibody fragment; or a multispecific antibody comprising a first variable region that recognizes TGF-β3 and a second variable region that recognizes a B cell in the manufacture of a suppressor of B cell activation; and
[16] use of TGF-β3; a molecule in which TGF-β3 is linked with an antibody or an antibody fragment; or a multispecific antibody comprising a first variable region that recognizes TGF-β3 and a second variable region that recognizes a B cell in the manufacture of an agent for treating an autoimmune disease.

### Brief Description of the Drawings

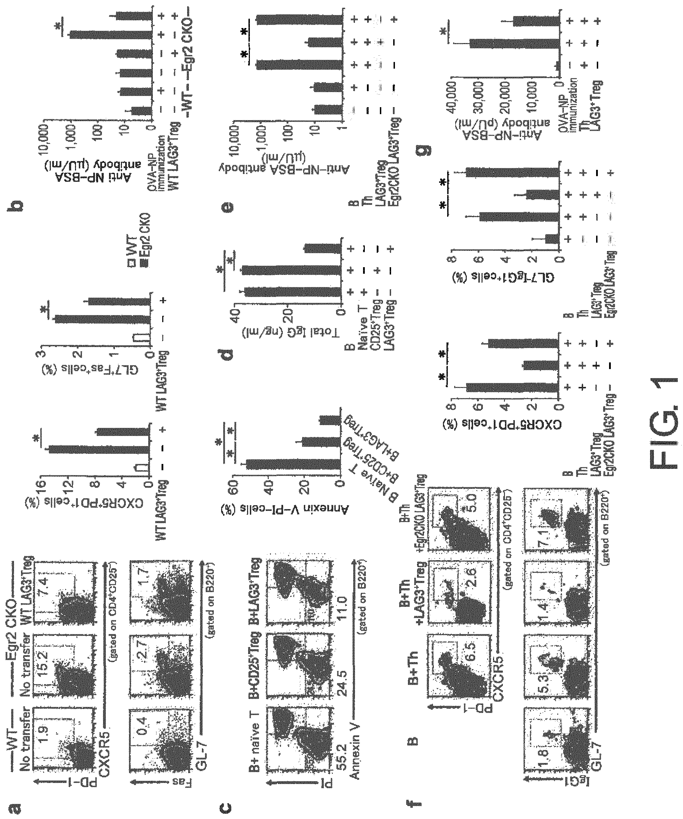

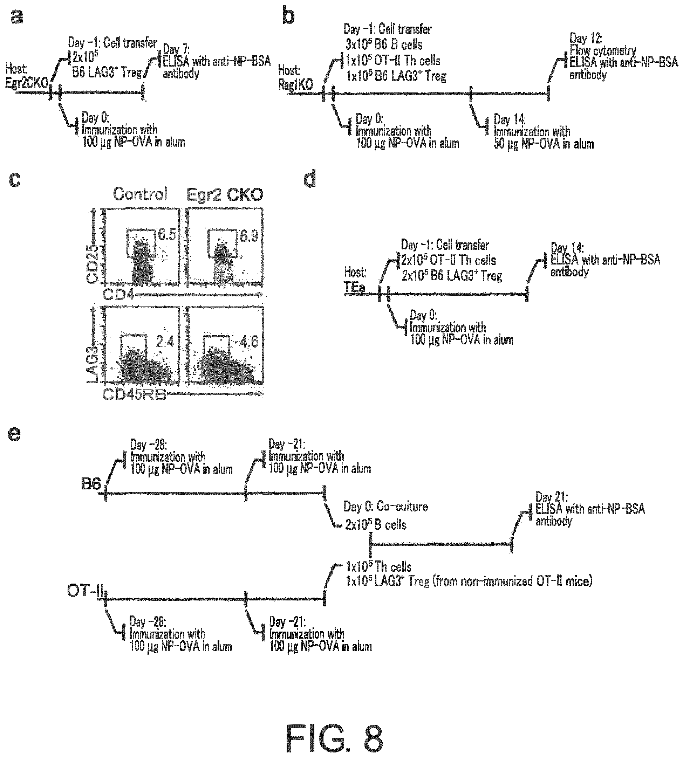

Fig. 1 shows Egr2-dependent control of antibody production by LAG3⁺ Treg. (a) Flow cytometry plots and quantification of CD4⁺CD25⁻CXCR5⁺PD-1⁺ T_{FH} and B220⁺GL-7⁺Fas⁺ GCB from wild type (WT) or Egr2CKO mice at day 7 with or without the adoptive transfer of WT CD4⁺CD25⁻LAG3⁺ regulatory T cells (LAG3⁺ Treg) (*n* = 5/group). (b) NP-specific antibody responses of Egr2CKO and WT mice immunized once with NP-OVA/alum with or without the adoptive transfer of WT LAG3⁺ Treg, as described in Fig. 8a (*n* = 6/group). (c,d) *In vitro* B cell suppression by LAG3⁺ Treg. Each T cell subset stimulated with an anti-CD3 mAb was co-cultured with stimulated B cells. (c) Live B220⁺ B cells were quantified by AnnexinV/PI staining 72 hours after anti-IgM stimulation (*n* = 3/group). (d) Total IgG was determined in anti-CD40 antibody/IL-4-stimulated B cell culture supernatants on day 7 by ELISA (*n* = 3/group). (e) *In vivo* NP-specific antibody responses. C57BL/6 (B6) B cells and OT-II CD4⁺CD25⁻LAG3⁻ helper T (Th) cells were transferred with or without B6 LAG3⁺ Treg or Egr2CKO LAG3⁺ Treg into Rag1KO mice immunized twice with NP-OVA/alum, as described in Fig. 8b (*n* = 6/group). (f) Flow cytometry plots and quantification of splenic CD4⁺CD25⁻CXCR5⁺PD1⁺ T_{FH} and B220⁺GL7⁺IgG1⁺ GCB from the same mice as in e. (g) B cell suppression by LAG3⁺ Treg in non-lymphopenic TEa mice, as described in Fig. 8d (*n =* 6/group). **P* < 0.05 (unpaired two-tailed Student's *t*-test). Data are representative of three independent experiments. The means ± standard deviation (s.d.) are shown.

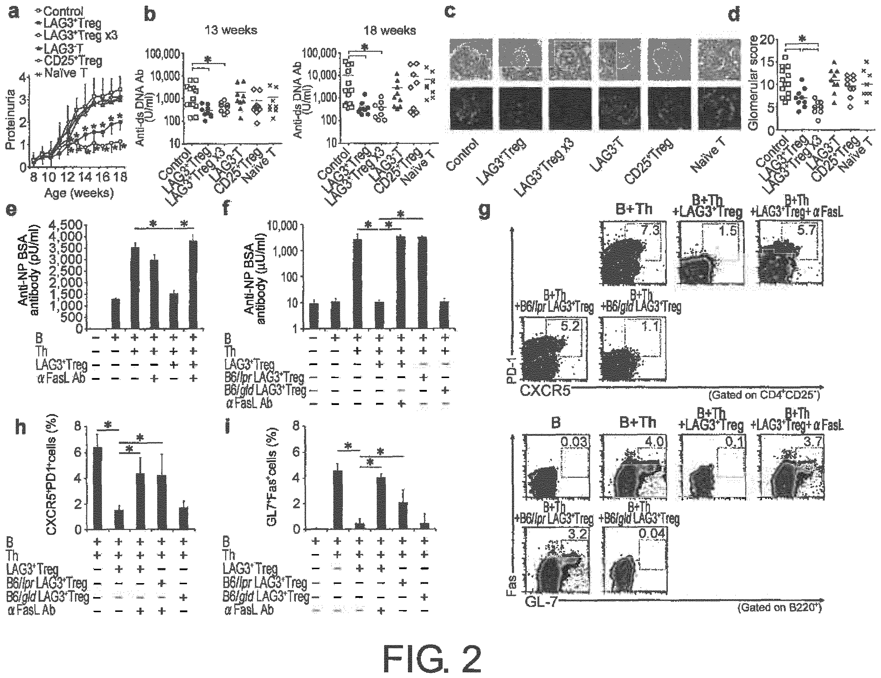

Fig. 2 shows the regulation by LAG3⁺ Treg of B cell functions through Fas. (a-d) Treatment of MRL/*lpr* mice with adoptive transfer of T cell subsets. Ten-week-old MRL/*lpr* mice were injected intravenously (*i.v*.) with LAG3⁺ Treg (*n* = 9), CD4⁺CD25⁻LAG3⁻ T cells (LAG3- T) (*n* = 9), CD4⁺CD25⁺ Treg (CD25⁺ Treg) (*n* = 9), or CD4⁺CD25⁻CD45RB^{high} T cells (naïve T) (*n* = 8) from MRL/+ mice (1 x 10⁵ cells each). The mice of LAG3⁺ Treg x3 group (*n* = 8) were injected with LAG3⁺ Treg (1 x 10⁵ cells) at 10 weeks of age followed by a weekly injection of the same amount of LAG3⁺ Treg for two times. The control group received PBS (*n* = 13). (a) Proteinuria progression. **P* < 0.05 *vs* control group. (b) Quantification of serum anti-ds DNA antibodies. (c) H&E staining (upper panels) and IgG immunofluorescent staining (lower panels) of kidney sections. Scale bars: 50 µm. (d) Glomerular scores. (e) LAG3⁺ Treg-mediated suppression of *in vitro* NP-specific antibody responses, as described in Fig. 8e *(n* = 6/group). (f) NP-specific antibody responses of Rag1KO mice injected with B6 B cells and OT-II Th cells with or without LAG3⁺ Treg from WT, B6/*lpr*, or B6/*gld* mice, as outlined in Fig. 1e (*n* = 6/group). An anti-FasL blocking antibody (200 µg/mouse) was injected *i.v*. weekly. (g-i) Flow cytometry plots (g) and quantification of splenic CD4⁺CD25⁻CXCR5⁺PD1⁺ T_{FH} (h) and B220⁺GL-7⁺Fas⁺ GCB (i) from the same mice as in f. Statistical significances in a and d were analyzed by the Mann-Whitney *U*-test, and others were analyzed by two-tailed Student's *t*-test (**P* < 0.05). The experiments in e and f were repeated three times. The means ± standard deviation (s.d.) are shown.

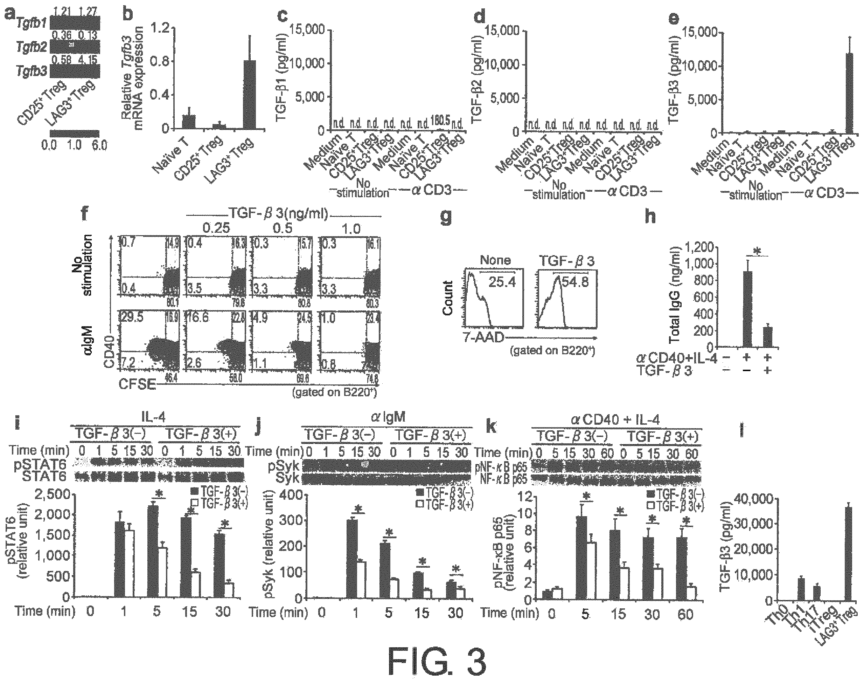

Fig. 3 shows the suppression by LAG3⁺ Treg of B cell activation through TGF-β3. (a) Microarray comparison of the gene expression profiles between B6 CD25⁺ Treg and B6 LAG3⁺ Treg. Normalized expression values from B6 CD4⁺CD25⁻CD45RB^{high} naïve T cells are shown according to the color scale. (b) The *Tgfb3* mRNA expression in sorted T cell subsets taken from the spleens of B6 mice (*n* = 3/group). (c-e) The TGF-β1, 2, and 3 protein levels in the culture supernatants of T cell subsets from B6 mice determined by ELISA. Cells were seeded at 1 x 10⁵ cells/well (*n* = 4/group). (f) CFSE-labeled B cells were stimulated with or without an anti-IgM mAb in the presence or absence of recombinant (r)TGF-β3 (*n* = 3/group). (g) Viability of the anti-IgM-stimulated B cells in the presence or absence of rTGF-β3 (1 ng/ml) was assessed by 7-AAD (*n* = 3/group). (h) The effects of TGF-β3 on total IgG production (measured in the same way as in Fig. 1d; *n* = 3/group) in the culture supernatants of anti-CD40 antibody/IL-4-stimulated B cells. (i-k) STAT6 (i), Syk (j), and NF-κB p65 (k) phosphorylation in stimulated B cells with or without rTGF-β3, calculated as the ratio of phosphorylated to total protein levels *(n =* 3/group). (1) The TGF-β3 protein levels in the culture supernatants of freshly isolated B6 LAG3⁺ Treg or naïve B6 CD4⁺ T cells cultured under Th0, Th1, Th2, or Th17 conditions determined by ELISA. Cells were seeded at 3 x 10⁵ cells/well (*n* = 3/group). **P <* 0.05 (unpaired two-tailed Student's *t*-test). The means ± standard deviation (s.d.) are shown.

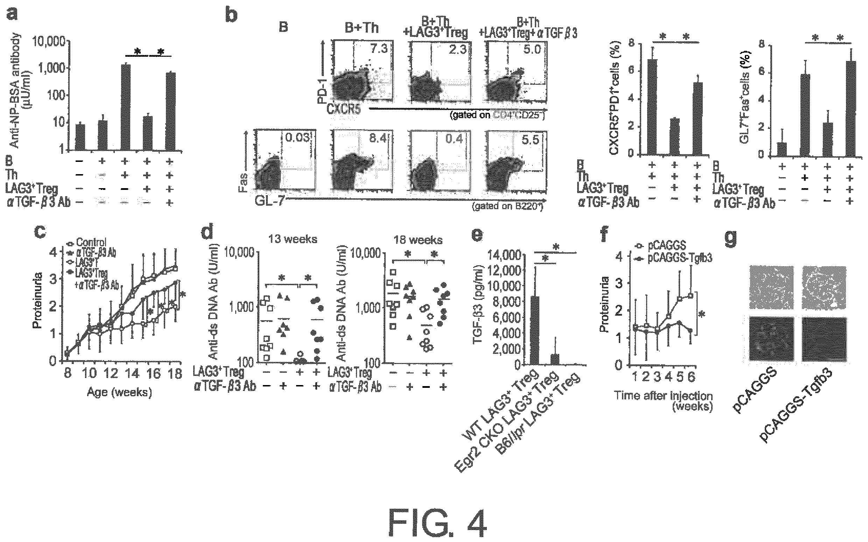

Fig. 4 shows the amelioration of lupus-like pathology by TGF-β3. (a) *In vivo* inhibition of LAG3⁺ Treg-mediated B cell suppression by weekly injections of an anti-TGF-β3 blocking mAb (100 µg/mouse) in NP-OVA immunized Rag1KO mice transferred with B cells and T cells, as outlined in Fig. 1e (*n* = 6/group). (b) Flow cytometry plots and quantification of splenic CD4⁺CD25⁻CXCR5⁺PD1⁺ T_{FH} and B220⁺GL-7⁺Fas⁺ GCB from the same mice as in a. (c,d) Proteinuria progression (c) and serum levels of an anti-dsDNA antibody (d) in MRL/+ LAG3⁺ Treg-transferred MRL/*lpr* mice with or without a weekly injection of an anti-TGF-β3 mAb (100 µg/mouse) (*n* = 8 mice/group). **P* < 0.05 *vs* LAG3⁺ Treg group. (e) Production of TGF-β3 by anti-CD3 antibody-stimulated LAG3⁺ Treg from WT, Egr2CKO, or B6/*lpr* mice, as in Fig. 3e *(n* = 6/group). (f) Proteinuria progression in MRL/*lpr* mice after *i.v*. injection with the pCAGGS control (n = 8) or pCAGGS-Tgfb3 plasmid vector (n = 7). (g) Kidney sections subjected to H&E staining (upper panels) and IgG immunofluorescent staining (lower panels) from the same mice as in f (representative images). Scale bars, 50 µm. Statistical significances in c and f were analyzed by the Mann-Whitney *U*-test, and others were analyzed by two-tailed Student's *t*-test (**P* < 0.05). The experiments in e were repeated three times. The means ± standard deviation (s.d.) are shown.

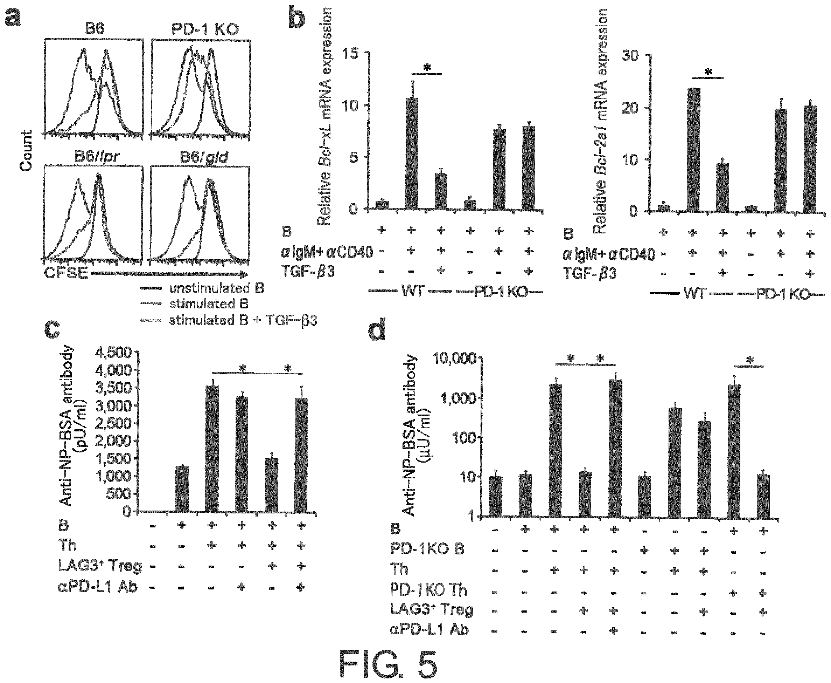

Fig. 5 shows the effect of PD-1 expression on B cells into the suppressive activity of LAG3⁺ Treg. (a) Resistance to TGF-β3-mediated B cell suppression in PD-1-deficient (PD-1KO) mice. CFSE-labeled B cells from B6, PD-1KO, B6/*lpr,* or B6/*gld* mice were stimulated *in vitro* for 72 hours with anti-IgM and anti-CD40 antibodies in the presence or absence of rTGF-β3 (1 ng/ml). The CSFE staining intensity of B220⁺ B cells (histograms). (b) *Bcl-xL* and *Bcl-2a1* mRNA levels in anti-IgM-stimulated B cells from WT or PD-1KO mice in the presence or absence of rTGF-β3 (1 ng/ml) (*n* = 3/group). (c)Inhibition of LAG3⁺ Treg-mediated suppression of *in vitro* NP-specific antibody responses by an anti-PD-L1 blocking mAb. The experimental procedure is outlined in Fig. 8e *(n* = 6/group). (d) NP-specific antibody responses of Rag1KO mice injected with B6 B cells and OT-II Th cells from B6 or PD-1KO mice with or without LAG3⁺ Treg from B6 mice. The anti-PD-L1 blocking antibody (200 µg/mouse) was injected *i*.*v*. every 3 days. The anti-NP-BSA antibody levels were determined as in Fig. 1e (*n* = 6/group). **P* < 0.05 (unpaired two-tailed Student's *t*-test). Data are representative of three independent experiments. The means ± standard deviation (s.d.) are shown.

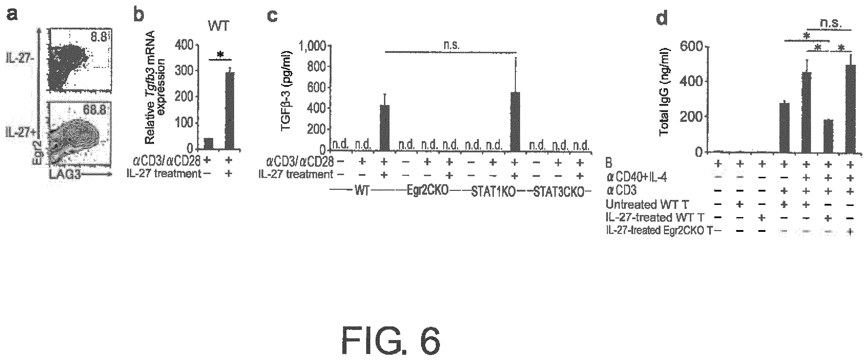

Fig. 6 shows IL-27-mediated induction of TGF-β3-producing Egr2⁺ regulatory T cells from naive T cells. (a) IL-27-mediated induction of Egr2 and LAG-3 expression on CD4⁺ T cells. Freshly isolated naive CD4⁺ T cells were stimulated with anti-CD3 mAb/CD28 mAb in the presence or absence of IL-27. Cells were stained for Egr2 and LAG-3 expression on day 5. (b) Quantitative RT-PCR analysis of the *Tgfb3* mRNA expression in naive WT CD4⁺ T cells activated as in a (day 3). (c) ELISA for TGF-β3 in culture supernatants of activated naive WT, Egr2CKO, STAT1KO, or *Stat3*^{*fl*/*fl*} CD4-*Cre*⁺ (STAT3CKO) CD4⁺ T cells as in a (day 5). (d) *In vitro* B cell suppression by IL-27-treated naive WT CD4⁺ T cells. IL-27-treated or untreated naïve WT or Egr2CKO CD4⁺ T cells stimulated with the anti-CD3 mAb were co-cultured with anti-CD40 antibody/IL-4-stimulated B cells. Total IgG was determined in culture supernatants on day 7 by ELISA (*n* = 3/group). **P* < 0.05 (unpaired two-tailed Student's *t*-test); n.d., not detected; n.s., not significant. Data are representative of three independent experiments. The means ± standard deviation (s.d.) are shown.

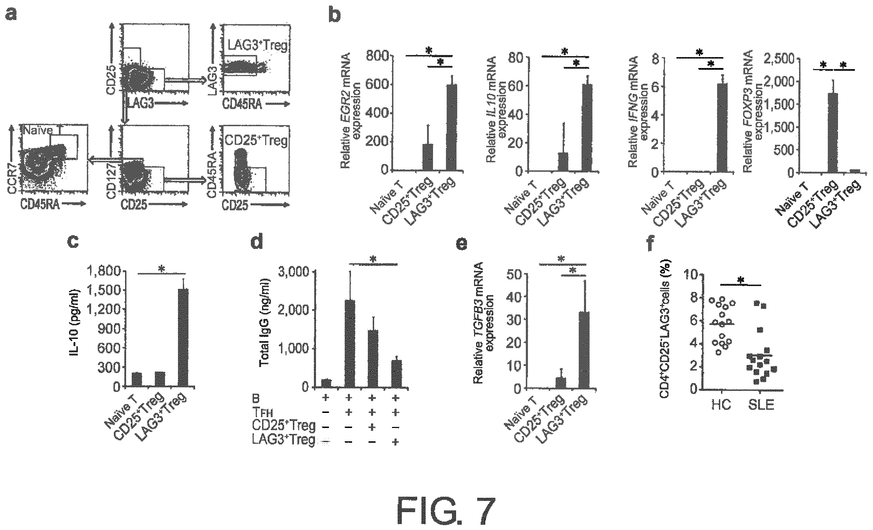

Fig. 7 shows suppression of antibody production by human CD4⁺CD25⁻D45RA⁻LAG3⁺ T cells. (a) FACS gating of CD4⁺CD25⁻CD127^{high}CCR7⁺ T cells (naive T), CD4⁺CD25^{high}CD127^{low}CD45RA⁻ T cells (CD25⁺ Treg), and CD4⁺CD25⁻CD45RA⁻LAG3⁺ T cells (LAG3⁺ Treg). Freshly isolated human peripheral blood mononuclear cells (PBMCs) from healthy controls (HC) were stained for CD4, CD25, CD45RA, CD127, CCR7, and LAG3, and the percentages of cells in each quadrant are indicated. (b) Quantitative RT-PCR analysis of *EGR2, IL10*, *IFNG,* and *FOXP3* mRNA expression in anti-CD3 mAb-stimulated conditions for each CD4⁺ T cell subset from HC (n = 3/group). (c) The IL-10 protein levels in the culture supernatants on day 7 of the indicated T cell subsets (determined by ELISA) (n = 3/group). (d) *In vitro* B cell suppression by each CD4⁺ T cell subset from HC. Each CD4⁺ T cell subset was co-cultured with T_{FH} and Staphylococcal enterotoxin B (SEB)-stimulated B cells. Total IgG was determined in culture supernatants by ELISA (n = 3/group). (e) The *TGFB3* mRNA expression in sorted T cell subsets collected from HC (*n* = 3). (f) Percentages of CD4⁺CD25⁻CD45RA⁻LAG3⁺ T cells in each HC (*n* = 15) and systemic lupus erythematosus patients (SLE) (*n* = 15) as in a. **P* < 0.05 (unpaired two-tailed Student's *t*-test). The means ± standard deviation (s.d.) are shown.
Fig. 8 shows that LAG3⁺ Treg-mediated regulation of germinal center B cells and follicular helper T cells is Egr2-dependent. (a) Diagrammatic representation of the experimental protocol for the NP-specific antibody responses of WT mice and *Egr2*^{*fl*/*fl*} CD4-*Cre*⁺ (Egr2CKO) mice immunized once with 100 µg NP-OVA/alum with or without adoptive transfer of WT LAG3⁺ Treg. The serum levels of the anti-NP-BSA antibody were determined by ELISA 7 days after immunization. (b) Diagrammatic representation of the experimental protocol for cell transfer into Rag1-deficient (Rag1KO) mice immunized twice with NP-OVA/alum. C57BL/6 (B6) B cells and OT-II CD4⁺CD25⁻LAG3⁻ helper T (Th) cells were injected intravenously (*i.v*.) into Rag1KO mice in combination with or without LAG3⁺ Treg from B6 mice 1 day before the intraperitoneal (*i*.*p*.) immunization with NP-OVA/alum, and given a booster immunization 14 days after the primary immunization. The anti-NP-BSA antibody in sera was measured by ELISA 7 days after the booster immunization. (c) Splenic LAG3⁺ Treg in Egr2CKO mice. Dot plots were gated on CD4⁺ T cells (upper panels) and CD4⁺CD25⁻ T cells (lower panels). The numbers indicate the percentage (%) of cells contained within the rectangular regions. The data are representative of four independent experiments. (d) Diagrammatic representation of the experimental protocol for LAG3⁺ Treg transfer into TEa TCR transgenic mice. LAG3⁺ Treg from B6 mice and OT-II Th cells were injected intravenously (*i.v*.) into TEa mice and subsequently immunized with NP-OVA/alum once. The anti-NP-BSA antibody levels were determined by ELISA. (e) *In vitro* NP-specific antibody production. B cells and Th cells purified from NP-OVA/alum-pre-immunized B6 mice and OT-II mice, respectively, were incubated with or without LAG3⁺ Treg from non-immunized OT-II mice in the presence or absence of an anti-FasL blocking antibody, and the anti-NP-BSA antibody in the sera was measured by ELISA.

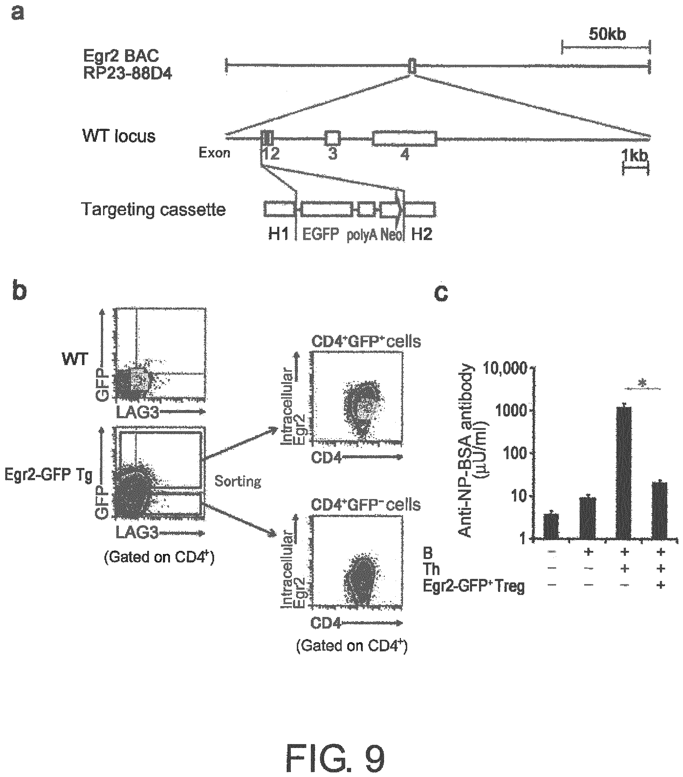

Fig. 9 shows the regulatory activity of CD4⁺CD25⁻Egr2-GFP⁺ T cells from Egr2-GFP transgenic mice. (a) A schematic representation of the method for producing Egr2-GFP transgenic mice (Egr2-GFP mice) using an Egr2 BAC clone (RP23-884D). A part of the *Egr2* coding region was replaced by the EGFP-SV40 poly A cassette. H1 and H2, homology arms; Neo, neomycin-resistant gene. (b) The GFP expression in Egr2-GFP mice. The strategy of sorting CD4⁺GFP⁺ and CD4⁺GFP⁻ cells from CD4⁺ cells from Egr2-GFP mice, and evaluation of the egr2 protein expression in sorted cells. Sorted T cells were stained intracellularly with an anti-Egr2 monoclonal antibody (mAb). Data are representative of three independent experiments. (c) Examination of CD4⁺CD25⁻Egr2-GFP⁺ T cell-mediated *in vitro* B cell suppression. The NP-specific antibody responses of Rag1KO mice injected with B6 B cells and OT-II Th cells in combination with or without CD4⁺CD25⁻Egr2-GFP⁺ T cells (Egr2-GFP⁺ Treg) from Egr2-GFP mice (*n* = 6 per group). The anti-NP antibody levels were determined as in Fig. 1 e. Statistical analyses were performed using unpaired two-tailed Student's *t*-test (**P* < 0.05). The means ± standard deviation (s.d.) are shown.

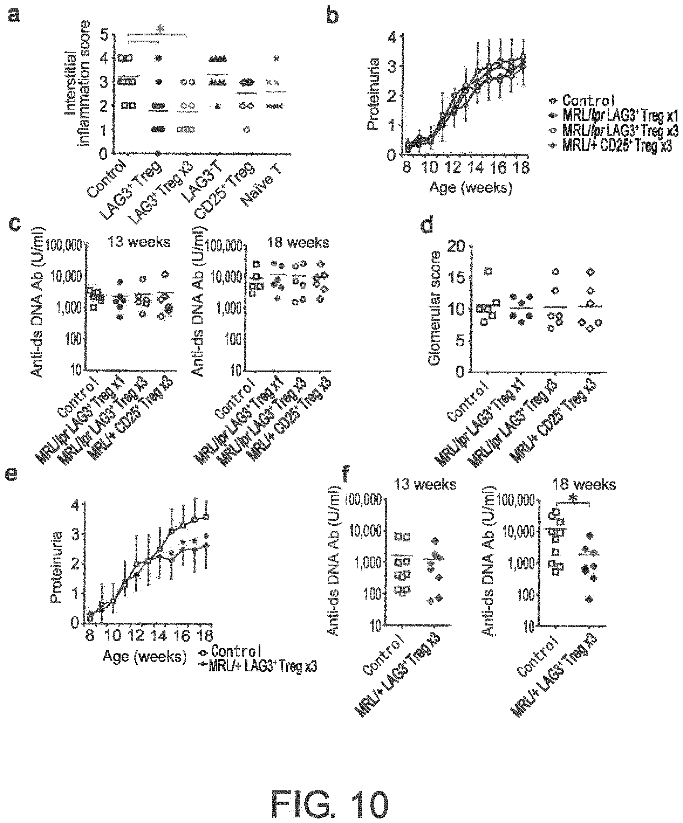

Fig. 10 shows the mechanism of disease regulation by LAG3⁺ Treg in systemic lupus erythematosus murine models, and therapeutic effects thereof. (a) Interstitial inflammation scores of kidney sections. At 10 weeks of age, MRL/*lpr* mice in the treatment group were injected intravenously (*i.v.*) with CD4⁺CD25⁻LAG3⁺ Treg (LAG3⁺ Treg) (*n* = 9), CD4⁺CD25⁻LAG3⁻ T cells (LAG3- T) (*n* = 9), CD4⁺CD25⁺ Treg (CD25⁺ Treg) (*n* = 9), or CD4⁺CD25⁻CD45RB^{high} T cells (naive T) (*n* = 8) from MRL/+ mice (1 x 10⁵ cells each). In the LAG3⁺ Treg x3 group *(n =* 8), mice were first injected with LAG3⁺ Treg (1 x 10⁵ cells) at 10 weeks of age followed by a weekly injection for two times. Mice in the control group received PBS (*n* = 13). The data source is identical to that in Fig. 2a-d. Interstitial inflammation in each kidney section was graded by standard methods, as described in the "Methods" section. (b-d) Results of assays performed with MRL/*lpr* mice administered with LAG3⁺ Treg from MRL/*lpr* mice or CD25⁺ Treg from MRL/+ mice *(n =* 6 per group). All other conditions were identical to those in Fig. 10a, except that LAG3⁺ Treg from MRL/*lpr* mice (x1 and x3) and CD25⁺ Treg from MRL/+ mice (x3) were used. *n* = 6 mice per group. The levels of proteinuria (b), anti-ds DNA antibody (c), and glomerular scores (d) were examined using the same methods as in Fig. 2a-d. The means ± standard deviation (s.d.) are shown. (e,f) Three time injections of LAG3⁺ Treg after the onset of overt proteinuria. MRL/*lpr* mice were first injected with LAG3⁺ Treg from MRL/+ mice (1 x 10⁵ cells) at 13 weeks of age followed by a weekly injection (MRL/+ LAG3⁺ Treg x3) for two times. Mice in the control group received PBS. The levels of proteinuria (e) and anti-ds DNA antibody (f) were examined using the same methods as in Fig. 2a,b (Control, *n* = 10; LAG3⁺ Treg x3, *n* = 8). Statistical significances in a, d, and e were analyzed by the Mann-Whitney *U*-test, and others were analyzed by two-tailed Student's *t*-test (**P* < 0.05). The means ± standard deviation (s.d.) are shown.

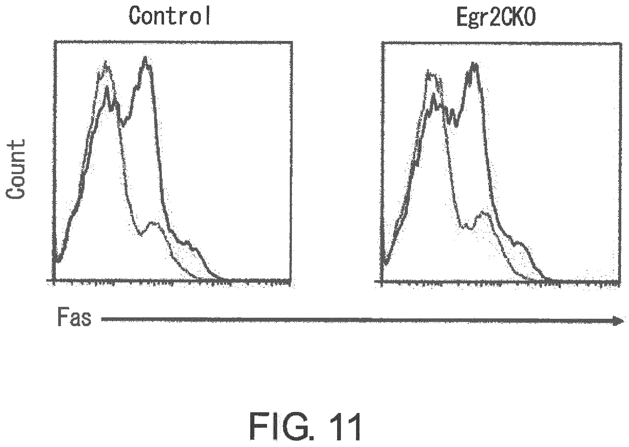

Fig. 11 shows the expression of Fas in Egr2CKO mice. Freshly isolated splenic CD4⁺ T cells from WT (*Egr2*^{*fl*/*fl*} CD4-*Cre⁻,* left panel) and Egr2CKO mice (*Egr2*^{*fl*/*fl*} CD4-*Cre*⁺, right panel) were incubated in anti-CD3 mAb-coated 96-well culture plates at 1 x 10⁵ cells/well for 72 hours. The Fas expression in LAG3 positive cells (black line) or LAG3 negative cells (grey line) within CD4⁺ T cells is shown as histograms. Representative data from three independent experiments are shown.

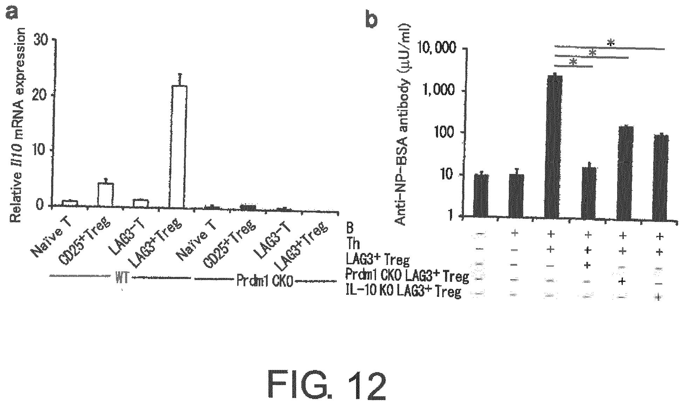

Fig. 12 shows LAG3⁺ Treg in Prdm1CKO and IL-10KO mice. (a) The *Il10* mRNA expression by sorted T cell subsets taken from the spleens of WT B6 or Prdm1CKO mice (*Prdm1*^{*fl*/*fl*} CD4-*Cre*⁻). The four distinct T cell subsets are as follows: (naïve T) CD4⁺CD25⁻LAG3⁻CD45RB^{high} cells; (CD25⁺ Treg) CD4⁺CD25⁺LAG3⁻ cells; (LAG3⁻ T) CD4⁺CD25⁻LAG3⁻ T cells; (LAG3⁺ Treg) CD4⁺CD25⁻LAG3⁺ cells (n = 3 per group). (b) The NP-specific antibody responses of Rag1KO mice injected with B6 B cells and OT-II Th cells in combination with or without LAG3⁺ Treg from WT B6, Prdm1CKO or IL-10KO mice (*n* = 5 per group). The anti-NP-BSA antibody levels were determined as in Fig. 1e. Statistical analyses were performed using unpaired two-tailed Student's *t*-test (**P* < 0.05). Data are representative of three independent experiments. The means ± standard deviation (s.d.) are shown.

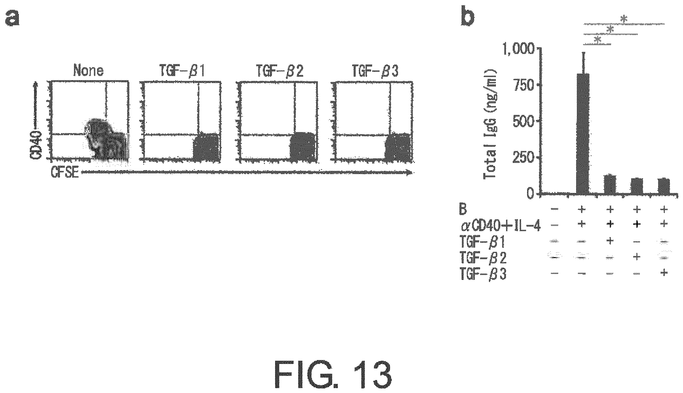

Fig. 13 shows suppression of B cell activation mediated by TGF-β1, 2, and 3. (a) CFSE-labeled splenic B cells from naive B6 mice were stimulated *in vitro* for 72 hours with an anti-IgM mAb in the presence or absence of rTGF-β1 (1 ng/ml), rTGF- β2 (1 ng/ml), or rTGF- β3 (1 ng/ml). Dot plots show CFSE and CD40 expressions on B220⁺ B cells. (b) The effects of rTGF-β1, 2, or 3 on total IgG production in the culture supernatants of anti-CD40 antibody/IL-4-stimulated B cells. Total IgG levels were determined as in Fig. 1d (*n* = 3 per group). Data are representative of three independent experiments (*n* ≥ 3 mice per group). Statistical analyses were performed using unpaired two-tailed Student's *t*-test (**P* < 0.05). Data are representative of three independent experiments. The means ± standard deviation (s.d.) are shown.

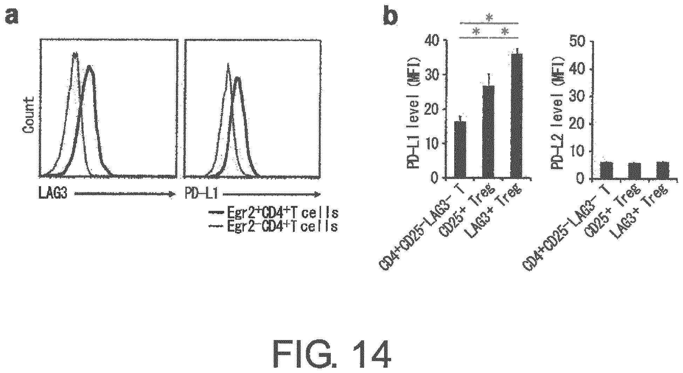

Fig.14 shows that LAG3⁺ Treg express PD-L1. (a) Effect of co-expression of LAG3 and PD-L1 on CD4⁺CD25⁻Egr2⁺ T cells from the spleens of B6 mice. Freshly isolated splenic CD4⁺CD25⁻ T cells were analyzed for the expression of LAG3 (left panel) and PD-L1 (right panel) according to their intracellular Egr2 expression. (b) Freshly isolated splenocytes from B6 mice were stained with an anti-CD4 mAb, anti-CD25 mAb, anti-LAG3 mAb, and PD-L1 mAb or PD-L2 mAb. The mean fluorescent intensity (MFI) levels of PD-L1 (left panel) and PD-L2 (right panel) in CD4⁺CD25⁻LAG3⁻ T cells, CD25⁺ Treg, and LAG3⁺ Treg are shown (*n* = 4 per group). Statistical analyses were performed using unpaired two-tailed Student's *t*-test (**P* < 0.05). Data are representative of three independent experiments. The means ± standard deviation (s.d.) are shown.

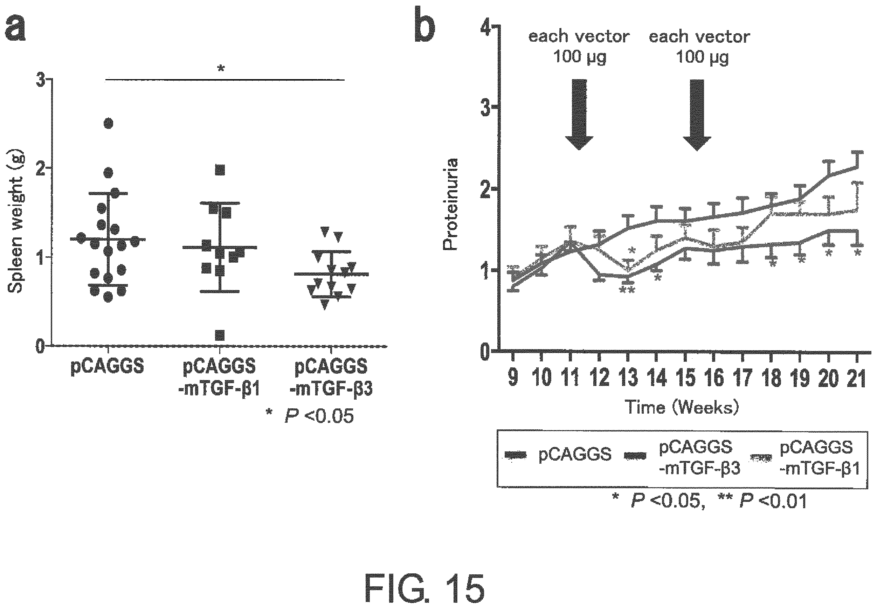

Fig. 15 compares the therapeutic effects of TGF-β1 and TGF-β3 in SLE-model mice. Full-length latent TGF-β1 and full-length latent TGF-β3 cloned into pCAGGS plasmids and the pCAGGS plasmid as control were each administered at 100 µg to MRL/*lpr* mice through the caudal vein. Each plasmid was administered twice, at 11 weeks and 15 weeks of age, and subsequently various pathological parameters were measured. The spleen weights (a) and proteinuria levels (b) were measured.

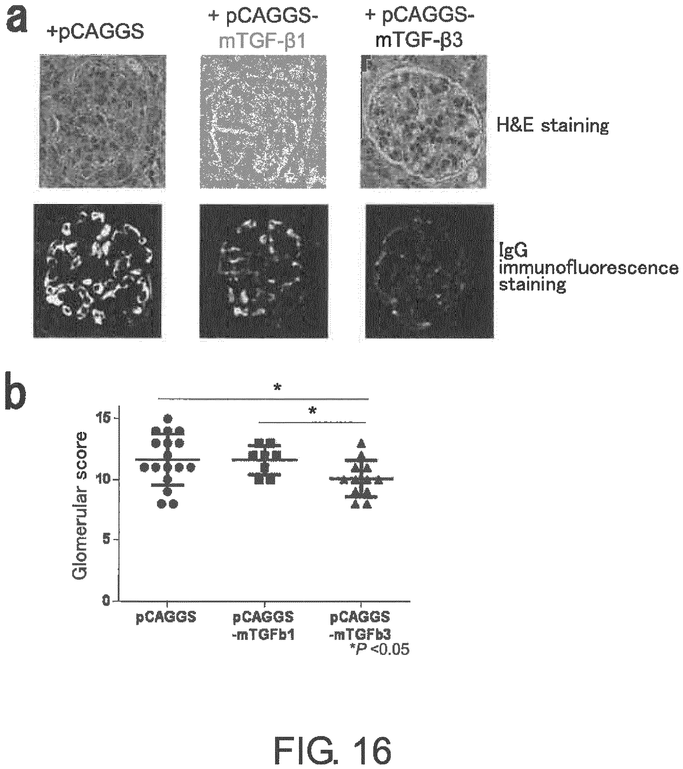

Fig. 16 compares the therapeutic effects of TGF-β1 and TGF-β3 in SLE-model mice (same as in the experiment of Fig. 15). Representative images of kidney sections subjected to H&E staining (upper panels) and IgG immunofluorescent staining (lower panels) are shown (a). The glomerular score levels were also determined.

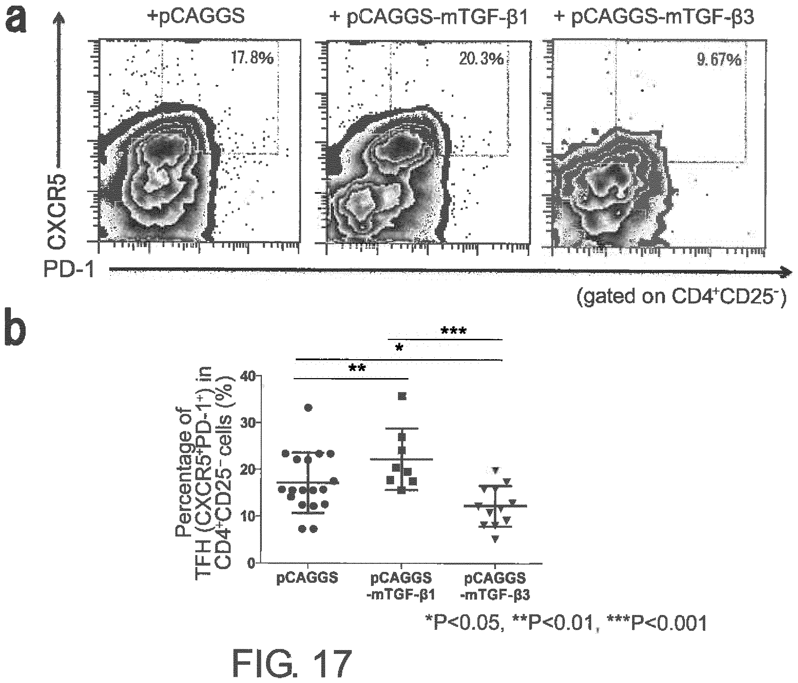

Fig. 17 compares the therapeutic effects of TGF-β1 and TGF-β3 in renal glomeruli of SLE model mice using histological scoring (same as the experiment of Fig. 15). The percentage of CD4/CXCR5/PD-1-positive CD25-negative follicular helper T cells (T_{FH}) was determined.

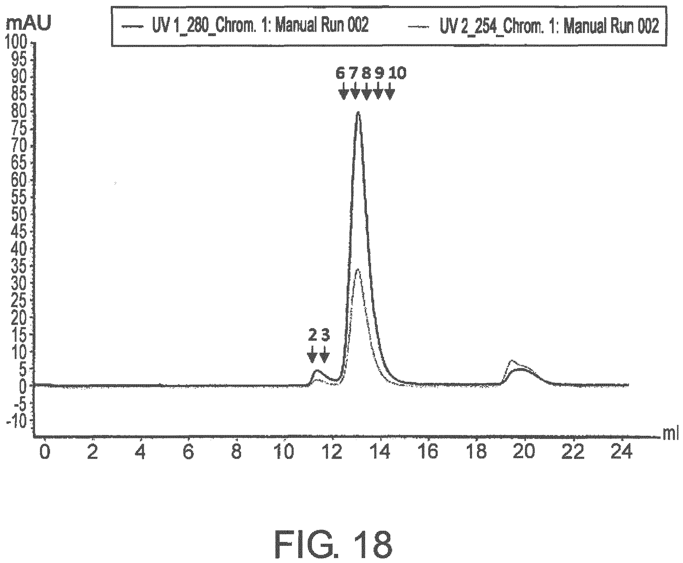

Fig. 18 shows a gel filtration chromatogram of latent TGF-β3.

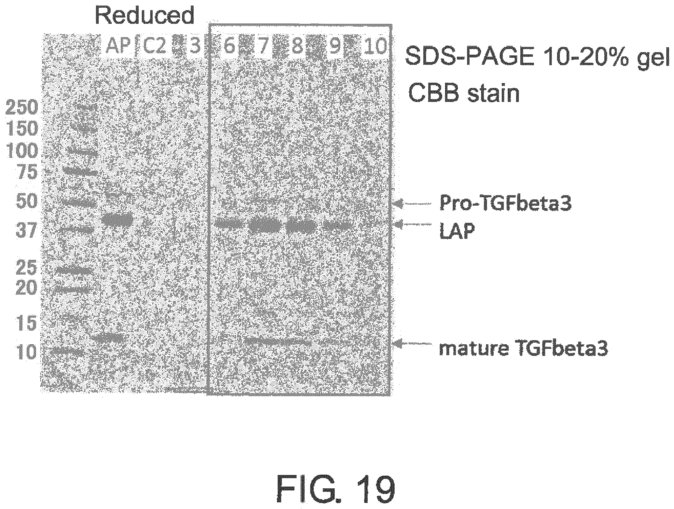

Fig. 19 shows an electrophoresis image of the gel filtration fractions of latent TGF-β3.

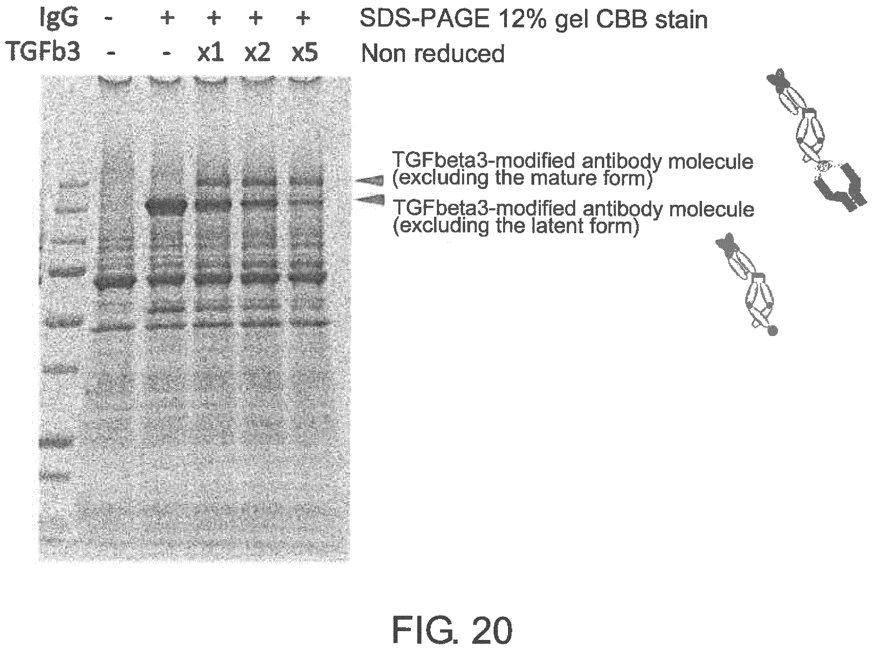

Fig. 20 shows an electrophoresis image of expression products of TGF-β3-modified antibody molecules.

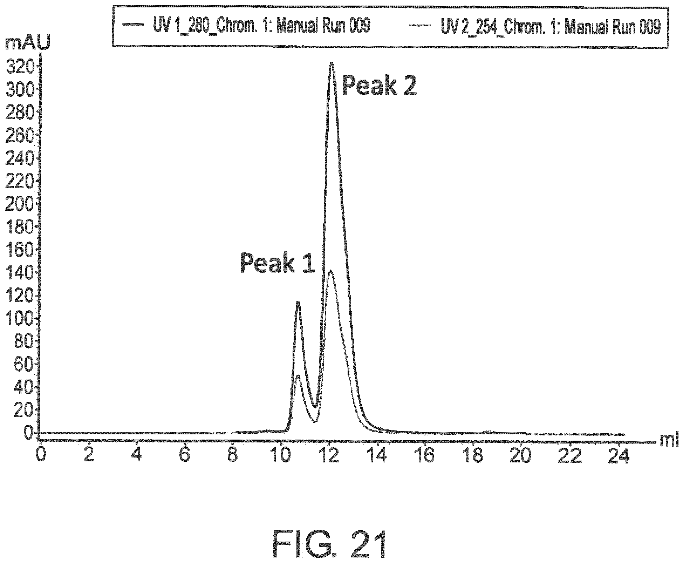

Fig. 21 shows a gel filtration chromatogram of TGF-β3-modified antibody molecules.

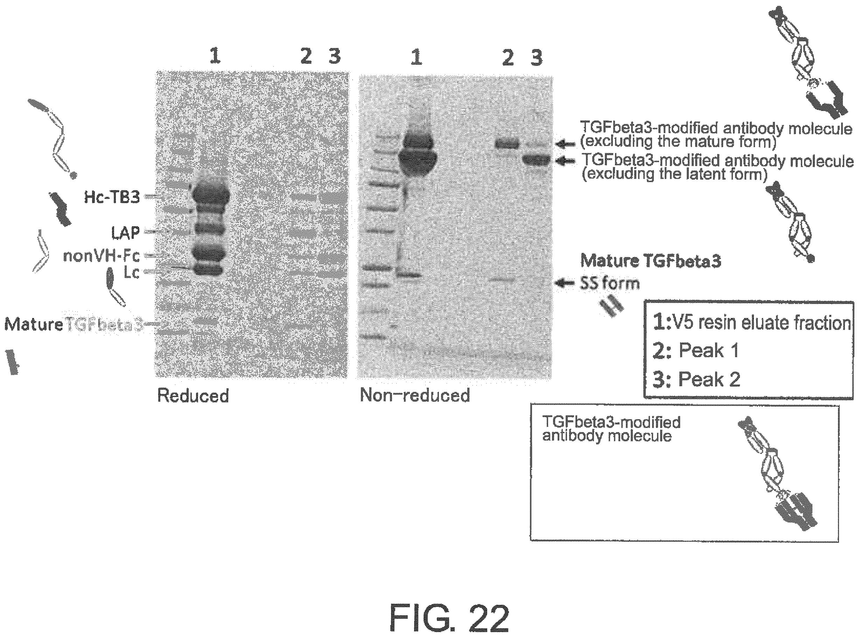

Fig. 22 shows an electrophoresis image of gel filtration fractions of TGF-β3-modified antibody molecules.

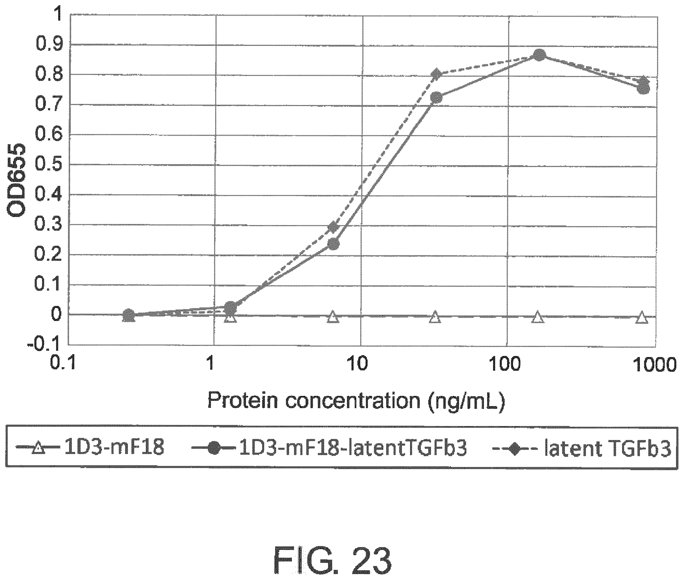

Fig. 23 shows the results of confirming the biological activities of TGF-β3-modified antibody molecules using HEK-Blue_TGFb cells. Each of the stimulating proteins and 5 ng/mL of Plasmin protease (PLN) were added to 50000 HEK-Blue_TGFb cells, the cells were cultured at 37°C in a CO₂ incubator for 20 hours, then the cell supernatant was collected, and the amount of secreted alkaline phosphatase was determined using the Quanti-Blue substrate by VersaMax spectrometer (OD655 nm).

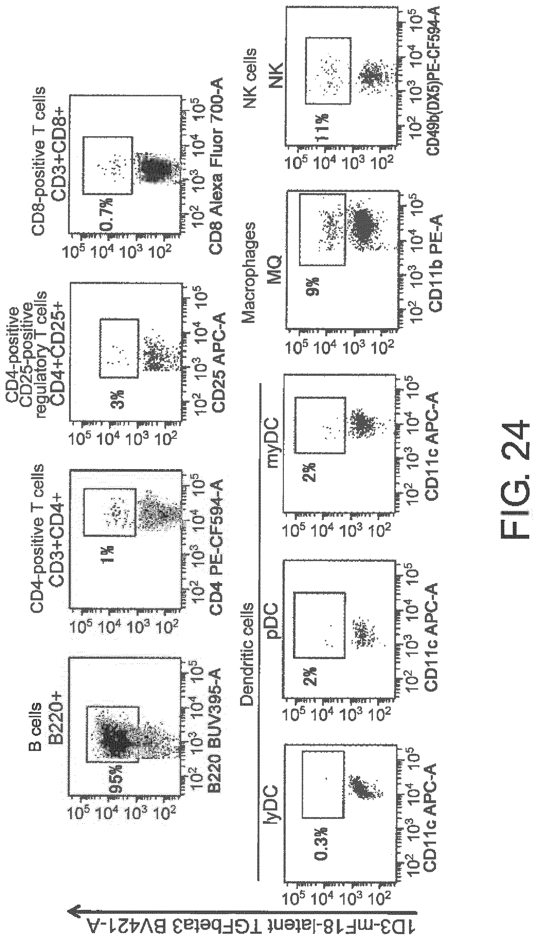

Fig. 24 shows the results of verifying the B cell-binding specificity of the TGF-β3-modified molecule. Splenocytes were subjected to reaction with various cell surface marker-detecting antibodies and TGF-β3-modified antibody molecules, and then the binding specificity of the TGFb3-modified antibody molecules was verified using BD LSRFortessaX-20. A V5 tag is inserted into the TGFb3-modified antibody molecules, and the TGFb3-modified antibody molecules were detected using a rabbit anti-V5 antibody and a BV421-labeled anti-rabbit IgG antibody. The lyDC (lymphoid DC) is CD11c^{hi}, CD11b^{-/lo}, and B220⁻; pDC (plasmacytoid DC) is CD11c^{lo}, CD11b⁻, Gr-1⁺, and B220+; myDC (myeloid DC) is CD11^{hi}, CD11b^{hi}, and B220⁻; MQ (macrophage) is CD11b^{hi} and CD11c⁻; and the NK (natural killer) cell is CD11b^{int}, CD11c⁻, and CD49b⁺.

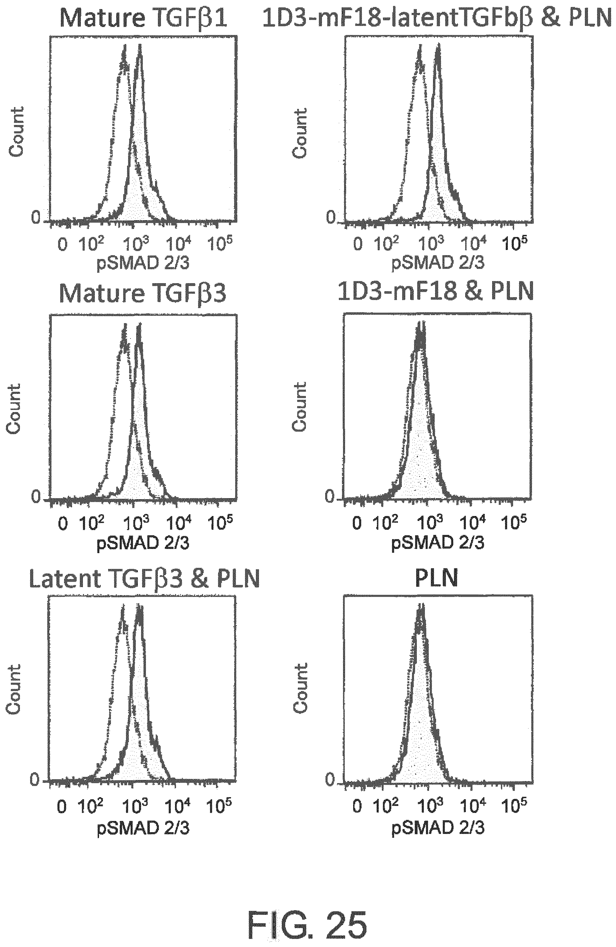

Fig. 25 shows the Smad signal activation by the TGF-β3-modified molecules. Each of the stimulating proteins and/or 5 ng/mL of Plasmin protease (PLN) were added to mouse splenocytes and cultured for 30 minutes, then the cells were fixed, and after membrane permeation treatment, they were subjected to reaction with an anti-B220 antibody and an anti-pSmad2/3 antibody (dashed lines indicate histograms obtained without stimulation).

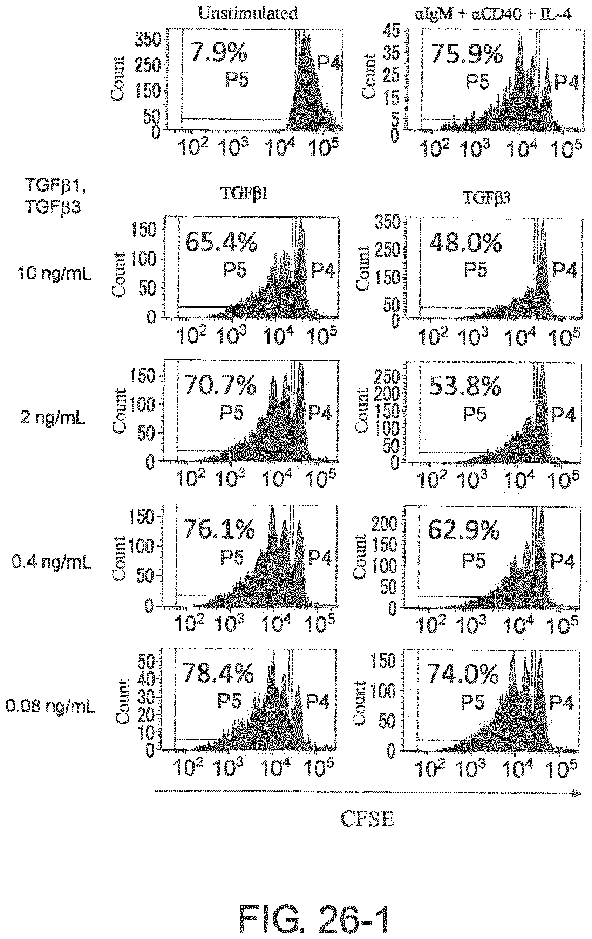

Fig. 26-1 shows the inhibitory effects on B-cell proliferation of TGF-β1 and TGF-β3. B cells were purified from mouse spleen using autoMACS and subjected to CFSE labeling. Each of the stimulating proteins was added and cells were cultured for three days. Then, dead cells were removed with 7AAD and the fluorescence intensity of CFSE in viable cells was determined using a FACS analyzer.

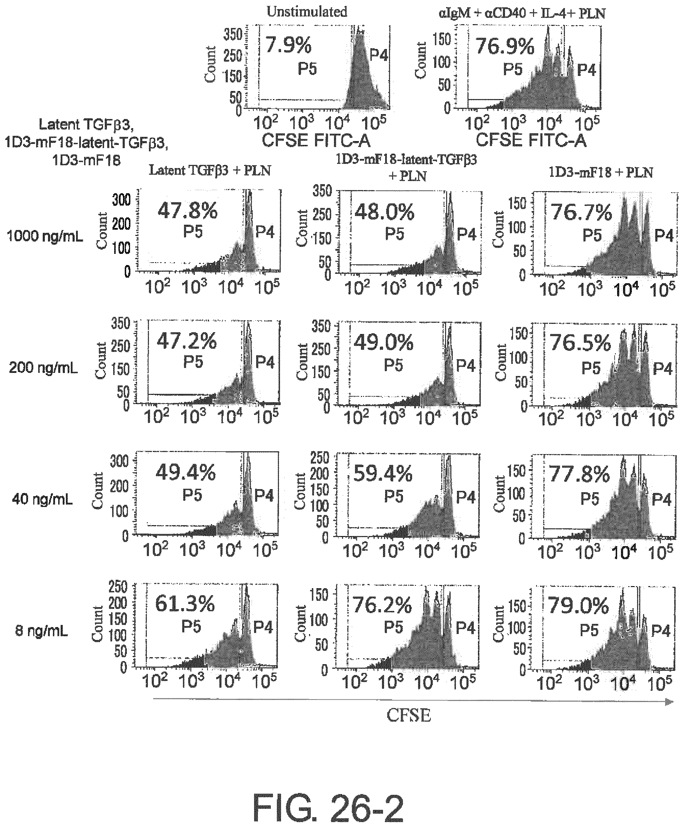

Fig. 26-2 shows the inhibitory effects on B-cell proliferation of the TGF-β3-modified molecules. B cells were purified from mouse spleen using autoMACS and subjected to CFSE labeling. Each of the stimulating protein and 0.5 µg/mL of Plasmin protease (PLN) were added and cells were cultured for three days. Then, dead cells were removed with 7AAD and the fluorescence intensity of CFSE in viable cells was determined using a FACS analyzer.

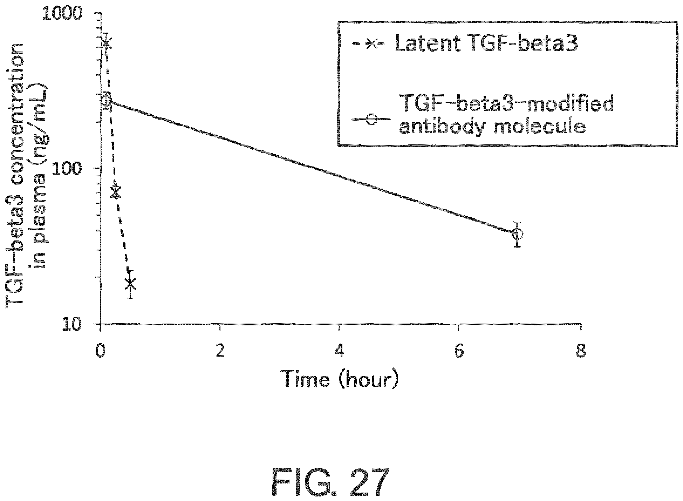

Fig. 27 shows the change in plasma TGF-b3 concentration when latent TGF-β3 or the TGF-β3-modified antibody molecule is intravenously administered at 1 mg/kg to mice (n = 3; the error bars show the standard deviation).

### Mode for Carrying Out the Invention

The present invention provides suppressors of B cell activation comprising TGF-β3. B cells are preferably autoreactive B cells, and examples of B cell activation suppression include suppression of antibody production by B cells. The suppressors can be used as therapeutic agents for autoimmune diseases.

TGF-β3 includes those having an amino acid sequence shown in SEQ ID NOs: 1 to 6; those having an amino acid sequence produced by partial alteration or modification of these amino acid sequences and having the same B cell activation suppressing activity, and also the latent and mature forms. Examples of the autoimmune diseases include systemic lupus erythematosus (SLE), pemphigus, multiple sclerosis, neuromyelitis optica (NMO), ANCA-associated vasculitis, rheumatoid arthritis, organ transplant rejection, Sjogren's syndrome, juvenile dermatomyositis, myasthenia gravis, and autoimmune thyroid disease including Graves' disease and Hashimoto's thyroiditis.

From another viewpoint, the present invention provides therapeutic agents for autoimmune diseases, which comprise a molecule formed by linking TGF-β3 with an antibody or an antibody fragment. An antibody fragment indicates a fragment produced by separating antibody components such as the variable region or the Fc region.

The antibody or antibody fragment may have a configuration in which a variable region that recognizes B cells is contained. B cells can be recognized using, for example, any one or more of CD19, CD20, CD40, CD22, IL21R, BAFF-R, BCMA, TACI, CD27, and CD138 as markers.

From another viewpoint, the present invention provides therapeutic agents for autoimmune diseases, which comprise a multispecific antibody comprising a first variable region that recognizes TGF-β3 and a second variable region that recognizes B cells.

From another viewpoint, the present invention provides therapeutic agents for autoimmune diseases, which comprise a molecule formed by linking TGF-β3 with PEG, a polysaccharide, or an artificial polypeptide, or a molecule formed by glycosylation of TGF-β3.

From another viewpoint, the present invention provides medical kits comprising the aforementioned therapeutic agents.

All prior art documents cited in this description are incorporated herein by reference.

### Examples

Herein below, the present invention will be specifically described with reference to the Examples, but it is not to be construed as being limited thereto. The accession number for the microarray data presented in the Examples is the number of E-MEXP-1343 [the ArrayExpress database].

### [Methods]

### Mice

C57BL/6 (B6), C57BL/6-Fas^{*lpr*/*lpr*} (B6/*lpr*), C57BL/6-FasL^{*gld*/*gld*} (B6/*gld*), MRL-Fas^{*lpr*/*lpr*} (MRL/*lpr*), and MRL-Fas^{+/+} (MRL/+) mice were purchased from Japan SLC. B6 recombinase-activating gene (*Rag*)-1-deficient (Rag1KO) mice, floxed-*Prdm1* (*Prdm1*^{*fl*/*fl*}) mice, *Il10*-deficient (IL-10KO) mice, T cell receptor (TCR) transgenic OT-II mice (specific for the chicken ovalbumin peptide (amino acid residues 323-339) in the context of MHC class II I-A^{b}) and TEa mice (specific for the Eα peptide (amino acid residues 52-68) from the MHC class II I-Eα molecule in the context of I-A^{b}) were purchased from Jackson Laboratories. Floxed-*Stat3* (*Stat3*^{*fl*/*fl*}) mice was purchased from Oriental Bio Service (Japan). Rag1KO mice were housed in microisolator cages with sterile filtered air. B6-*Pdcd1*-deficient (PD-1KO) mice (Non-Patent Document 47) were purchased from RIKEN BRC (Japan). Floxed *Egr2* (*Egr2*^{*fl*/*fl*}) mice were provided by Patrick Charnay (INSERM, France) (Non-Patent Document 48). *Egr2* conditional knockout (CKO) mice (*Egr2*^{*fl*/*fl*} CD4-*Cre*⁺), Prdm1 CKO mice (*Prdm1*^{*fl*/*fl*} CD4-*Cre*⁺), and STAT3 CKO (*Stat3*^{*fl*/*fl*} CD4-*Cre*⁺) were generated by crossing *Egr2*^{*fl*/*fl*} mice, *Prdm1*^{*fl*/*fl*} mice, or *Stat3*^{*fl*/*fl*} mice with CD4-Cre transgenic mice on a B6 background, respectively. *CD4-Cre* transgenic mice (line 4196) and *Stat1*-deficient (STAT1KO) mice were purchased from Taconic. Age-and sex-matched mice ≥ 7 weeks of age were used for all experiments. All animal experiments were approved by the ethics committee of the University of Tokyo Institutional Animal Care and Use Committee.

### Reagents, antibodies, and medium

The following reagents were purchased from BD Pharmingen: purified monoclonal antibody (mAb) for CD3ε (145-2C11), FasL blocking (MFL3), anti-CD40 (3/23), Fc block (anti-CD16/CD32), FITC anti-CD45RB (16A), FITC anti-Fas (Jo2), PE anti-CD45RB (16A), APC-Cy7 anti-CD45RB (16A), PE anti-LAG3 (C9B7W), APC anti-LAG-3 (C9B7W), FITC anti-IgG1 (A85-1), APC anti-IgG1 (A85-1), FITC anti-GL7 (Ly-77), FITC anti-CD25 (PC61), PE anti-CD25 (PC61), APC anti-CD25 (PC61), APC-Cy7 anti-CD25 (PC61), APC anti-CD4 (L3T4), APC-Cy7 anti-CD4 (L3T4), PE anti-CXCR5 (2G8), APC-Cy7 anti-B220 (RA3-6B2), PE anti-PD-1 (J43), biotinylated mAb for CD8a (53-6.7), CD19 (1D3), CD11c (HL3), CD45RB (16A), CD25 (7D4), and CXCR5 (2G8), streptavidin (SA)-FITC antibody (Ab), SA-APC, and SA-APC-Cy7. Alexa Fluor 488 anti-LAG3 mAb (C9B7W) and FITC anti-PD-L1 mAb (MIH6) were purchased from AbD Serotec. Qdot605 anti-CD4 mAb (RM4-5) and SA-Qdot605 were purchased from Invitrogen. PE anti-PD-L1 mAb (MIH5), PE anti-Egr2 mAb (erongr2), PE anti-PD-L1 mAb (10F. 9G2), and APC anti-B220 mAb (RA3-6B2) were purchased from eBioscience. NP(13)-OVA, and NP(9)-BSA were purchased from Biosearch Technologies. SA-conjugated microbeads were purchased from Miltenyi Biotec. FITC anti-mouse IgG Ab was purchased from Sigma. Anti-PD-L1 blocking mAb (10F.9G2)was purchased from Biolegend. Alexa 488 Fluor anti-GFP mAb was purchased from Medical & Biological Laboratories. Recombinant TGF-β1 (rTGF-β1) and rTGF-β3 were purchased from Miltenyi Biotec, and rTGF-β2, anti-TGF-β3 blocking polyclonal Ab (MAB234), and rIL-27 were purchased from R&D Systems.

For studies using human-derived cells, the following anti-human mAbs were used: V450 anti-hCD4 (RPA-T4), V500 anti-hCD4 (RPA-T4) (both from BD Biosciences), PerCP-Cy5.5 anti-human CD3 (UCHT1), Brilliant Violet 421 anti-hCD25 (BC96), APC anti-hCD19 (HIB19), PerCP-Cy5.5 anti-hCD4 (OKT4), Alexa Fluor 647 anti-hCD197 (G043H7), APC-Cy7 anti-human CD45RA (HI100) (all from BioLegend), Alexa Fluor 488 anti-hCD25 (BC96), and PE-Cy7 anti-hCD127 (eBioRDR5) (all from eBioscience). PE anti-hLAG3 polyclonal Ab was purchased from R&D Systems.

T cells and B cells were cultured in an RPMI-1640 medium supplemented with 10% FBS, 100 µg/ml L-glutamine, 100 U/ml penicillin, 100 µg/ml streptomycin, and 50 µM 2-mercaptoethanol (all purchased from Sigma).

### Generation of Egr2-GFP mice

The bacterial artificial chromosome (BAC) clone RP23-88D4, which contained the entire genomic *Egr2* locus, was obtained from BAC Libraries (Invitrogen). This clone was modified for the insertion of an enhanced green fluorescent protein gene (eGFP) with an SV40 polyA sequence at the initiation codon in Egr2 exon 1 using the Red/ET recombination system. The Egr2-eGFP construct linearized with PI-SceI was injected into the pronuclei of fertilized zygotes from B6 mice and transferred to pseudopregnant females. Offspring were screened for genomic integration by PCR of tail DNA using the following:
Egr2 promoter primer: forward 5'-AGACCGCATTTACTCTTATCACCAG-3' (SEQ ID NO: 7),
SV40polyA specific primer: reverse 5'- TGAGTTTGGACAAACCACAACTAGA-3' (SEQ ID NO: 8)
(PCR product size: 2.1 kb). Mice were generated by breeding F1 heterozygous transgenic males with wild-type (WT) females.

### Cell purification

Mouse spleens were cut into pieces and treated with collagenase type IV (Sigma-Aldrich). Red blood cells were lysed by hypotonic shock in an ammonium chloride with potassium (ACK) lysis buffer, followed by immediate isotonic restoration. Surface staining was performed in ice-cold PBS with 2% FCS in the presence of an FcR blocking antibody (anti-mouse CD16/CD32 mAb). To obtain highly purified CD4⁺ T cells, single-cell suspensions were first purified by negative selection with magnetic-activated cell sorting (MACS; Miltenyi Biotec) using anti-B220 mAb, anti-CD19 mAb, anti-CD11c mAb, and anti-CD8a mAb. To obtain highly purified CD4⁺CD25⁻CD45RB^{low}LAG3⁺ T cells, CD45RB^{high} cells were subsequently depleted with anti-CD45RB mAb. After FcR blocking, the prepared cells were stained with mAbs specific for CD4, CD25, CD45RB, and LAG3 in order to isolate CD4⁺CD25⁻CD45RB^{low}LAG3⁺ T cells (LAG3⁺ Treg), CD4⁺CD25 T cells (CD25⁺ Treg), CD4⁺CD25⁻LAG3⁻ helper T cells (Th cells) or CD4⁺CD25⁻CD62L^{hi}CD44^{low} (naïve T). Cells for intracellular anti-Egr2 staining were stained using the Foxp3 Staining Buffer Set (eBioscience) according to the manufacturer's protocol. The purities of MACS-sorted, and FACS (FACSVantage SE (Becton-Dickinson) or MoFlo XDP (Beckman Coulter))-sorted cells were > 90% and > 99%, respectively.

### Immunization

An NP-OVA alum solution was prepared by mixing NP(13)-OVA (Biosearch Technologies) in PBS solution with alum (Pierce) at a 1:1 ratio for 30 min at 4°C. The immunization with NP-OVA/alum was performed by *i.p.* injection.

### Adoptive transfer of LAG3⁺ Treg from WT mice into Egr2CKO mice

FACS-purified 2 × 10⁵ LAG3⁺ Treg from B6 mice were injected i.v. into 10-week-old Egr2CKO mice pre-immunized with or without 100 µg NP-OVA/alum 1 day prior to the cell transfer. The differentiation of T_{FH} and GCB in the spleen was analyzed by FACS 7 days after the cell transfer. The serum level of the anti-BSA-NP antibody was analyzed by ELISA 7 days after the immunization, as described above.

### B cell isolation and proliferation

Splenic B cells were purified by negative selection with MACS using a B cell isolation kit (Miltenyi Biotec) according to the manufacturer's protocol. The purity of MACS-sorted B cells was > 95% positive for B220 staining. B cells were labeled with 5 µM 5-(and 6-) carboxyfluorescein diacetate succinimidyl ester (CFSE; Dojindo) at 37°C for 10 min, and then stimulated with 10 µg/ml anti-IgM F(ab)'₂ (Jackson ImmunoResearch Laboratories) for 72 hours with or without rTGF-β1, 2, or 3 for B cell proliferation assays. Cells were stained with anti-B220 mAb, 7-Amino-Actinomycin D (7-AAD) (Biolegend) and PE anti-CD40 mAb. The percentages of viable 7-AAD-negative CFSE-diluted B220⁺CD40⁺ B cells and dead 7-AAD-positive B220⁺ cells were assessed by FACS.

### B cell activation co-culture assays

The wells of 96-well flat-bottomed plates were coated with 10 µg/ml of anti-CD3 mAb in 100 µl/well of PBS and incubated overnight at 4°C. The wells were washed, and MACS-purified B cells and each FACS-purified T cell subset (LAG3⁺ Treg, CD25⁺ Treg, or CD4⁺CD25⁻CD44^{lo}CD62L^{hi} naive T cells) or IL-27-treated CD4⁺ T cells described below were added into the coated wells at a density of 1 × 10⁵ cells/well in an RPMI medium containing 10 µg/ml of anti-CD40 mAb (3/23) + 10 µg/ml rIL-4 (Cell Signaling Technology) supplemented with or without rTGF-β3 (1 ng/ml). B cells undergoing apoptosis on day 3 and total IgG production in the culture supernatants on day 7 were determined using the Annexin V Apoptosis Detection Kit (BD Pharmingen) and a mouse IgG ELISA Quantitation Set (Bethyl Laboratories), respectively, according to the manufacturer's protocol.

### Adoptive transfer studies in Rag1KO mice

2 × 10⁵ of MACS-purified B cells from B6 or PD-1KO mice and 2 × 10⁵ of FACS-purified Th cells from OT-II or PD-1KO OT-II mice were injected *i.v*. into Rag1KO mice in combination with or without 1 × 10⁵ of FACS-purified LAG3⁺ Treg from B6, Egr2CKO, B6/*lpr*, B6/*gld*, or IL-10KO mice. Control mice received PBS. Mice were subsequently immunized with 100 µg of NP-OVA/alum 24 hours after the cell transfer. Mice were re-immunized with 50 µg of NP-OVA/alum 14 days after the first immunization. Where indicated, on the day after the cell transfer, mice were injected *i.v*. with an anti-FasL blocking antibody (200 µg/mouse) or an anti-TGF-β3 blocking antibody (100 µg/mouse) at weekly intervals, or an anti-PD-L1 blocking antibody (200 µg/mouse) every 3 days. The serum level of the anti-NP antibody was analyzed by ELISA, and splenocytes were analyzed by FACS 7 days after the re-immunization.

### Quantification of NP-specific antibody responses

The anti-NP IgG antibody levels were quantified by ELISA using NP(9)-BSA (Biosearch Technologies) as the capture antigen in the *in vitro* or *in vivo* antibody production assay. ELISA plates were prepared using the Immuno-Tek ELISA construction system (Zepto Metrix) according to the manufacturer's protocol. Following incubation with the sample serum or media, the plates were developed with an HRP-conjugated goat anti-mouse IgG1 and a TMB substrate. Serially diluted pooled sera from NP(13)-OVA immunized B6 mice were included as control on each plate. The concentration of the anti-NP IgG1 antibody was estimated by comparison with a standard curve constructed from the pooled sera.

### Adoptive transfer studies in TEa mice

2 × 10⁵ of FACS-purified Th cells from OT-II mice were injected *i.v*. into TEa mice in combination with or without 1×10⁵ of FACS-purified LAG3⁺ Treg from B6 mice. Control mice received PBS. Mice were subsequently immunized with 100 µg of NP-OVA/alum 24 hours after the cell transfer. The serum level of the anti-BSA-NP antibody was analyzed by ELISA 14 days after the immunization, as described above.

### Adoptive transfer studies in MRL/lpr mice

Eight-week-old MRL/*lpr* mice were randomly assigned to specific treatment groups. Ten-week-old MRL/*lpr* mice in the treatment group were injected *i.v*. with LAG3⁺ Treg, LAG3⁻T cells, CD25⁺ Treg, or naive T cells (1 x 10⁵ cells each) obtained from MRL/+ mice. The three-time injection group (MRL/+ LAG3⁺ Treg x3, MRL/+ CD25⁺ Treg x3, and MRL/*lpr* LAG3⁺ Treg x3) was adoptively transferred *i.v*. with 1 x 10⁵ LAG3⁺ Treg at weekly intervals (10, 11, and 12 or 13, 14, and 15 weeks of age, respectively; the mice were 10 weeks of age at the time of the first injection). Each T cell subset was first enriched by MACS and then sorted by FACS (based on the expression of CD4, CD25, CD45RB, and LAG3) as described above. Mice in the control group received PBS. Where indicated, on the day after the first cell transfer, mice were injected *i.v.* with an anti-TGF-β3 blocking antibody (100 µg/mouse) at weekly intervals. Mice were sacrificed at 18 weeks of age to examine pathological alterations. The anti-ds DNA antibody was measured using a mouse anti-ds DNA ELISA Kit (Shibayagi) at 13 and 18 weeks of age, according to the manufacturer's protocol.

### Urinary protein analysis

Proteins in the urine were assessed semiquantitatively using dip sticks (Albustix, Bayer) at weekly intervals (0 = none; 1= 30 to 100 mg/dl; 2 = 100 to 300 mg/dl; 3 = 300 to 1,000 mg/dl; 4 ≥ 1,000 mg/dl).

### Histological analysis

MRL/*lpr* mice were sacrificed at 18 weeks of age. Renal pathology was graded by standard methods for glomerular inflammation, proliferation, crescent formation, and necrosis as described in Non-Patent Document 49. The glomeruli were evaluated by examining at least 80 glomeruli per section by an examiner blind to the experimental conditions. Interstitial and tubular changes were also noted. Scores from 0 to 4 (where 0 represents "no damage" and 4 represents "severe") were assigned for each of these features.

### In vitro NP-specific antibody responses

B6 and OT-II mice were immunized with 100 µg of NP-OVA/alum. Mice were re-immunized with 100 µg NP-OVA/alum three weeks after the first immunization. 2 × 10⁵ of MACS-purified B cells from pre-immunized B6 mice and 1 × 10⁵ of FACS-purified CD4⁺CD25⁻LAG3⁻ helper T cells (Th cells) from pre-immunized OT-II mice were seeded in round-bottom 96-well plates 7 days after the re-immunization in combination with or without 1 x 10⁵ of FACS-purified LAG3⁺ Treg from non-immunized OT-II mice in the presence or absence of 10 µg/ml of an anti-PD-L1 blocking mAb (10F.9G2) or 10 µg/ml of an anti-FasL blocking mAb (MFL3). The culture supernatants were harvested at 3 weeks, and the anti-NP antibody level was analyzed by ELISA, as described above.

### RNA isolation, cDNA synthesis, and quantitative real-time PCR

Total T cell RNA was prepared using an RNeasy Micro Kit (Qiagen). RNA was reverse-transcribed into cDNA, and quantitative real-time PCR analysis was performed as described in Non-Patent Document 14. Relative RNA abundance was determined based on the abundance of control mouse β-actin or human GAPDH.

### DNA Microarray Analysis

Total RNA of CD4⁺CD25⁺, CD4⁺CD25⁻CD45RB^{low}LAG3⁺, and CD4⁺CD25⁻CD45RB^{high}LAG3⁻ FACS-purified T cells from B6 mice were harvested and then prepared for Affymetrix microarray analysis as described in Non-Patent Document 14. Microarray data were analyzed using Bioconductor (version 1.9) and statistical software R and GeneSpring GX version 7.3.1 (Silicon Genetics). All microarray data have been deposited in the ArrayExpress database (http://www.ebi.ac.uk/arrayexpress) under Accession No. E-MEXP-1343, which is identical to a previous study from the present inventors' laboratory (Non-Patent Document 14).

### Quantification of TGF-β family members

Each T cell subset was plated into anti-CD3 mAb or anti-CD3 mb/anti-CD28 mAb-coated wells at 3 x 10⁵ cells per well in a serum-free X-Vivo-20 medium (Lonza) for the determination of TGF-β1 and 2, or the RPMI medium described above for TGF-β3. All cultures were incubated at 37°C for 72 hours, and the supernatants were collected and stored at -80°C. TGF-β1, 2, and 3 levels in supernatants were determined by the TGF-β1 Emax ImmunoAssay System (Promega), TGF-β2 Quantikine ELISA Kit (R&D Systems), and TGF-β3 ELISA Kit (Mybiosource), respectively, according to the manufacturer's protocol. TGF-β3 levels in the RPMI medium supplemented with 10% FBS were lower than the minimum detectable level of the TGF-β3 ELISA Kit.

### Western blot analysis

MACS-purified B cells from B6 mice were pretreated with 0.75 µM CpG-ODN (ENZO Life Science) for 72 hours supplemented with or without rTGF-β3 (20 ng/ml) during the last 16 hours, and subsequently stimulated with 10 µg/ml of anti-CD40 mAb, 10µg/ml of rIL-4, or 10 µg/ml of anti-IgM F(ab)'₂ in the RPMI medium. Following the stimulation, cells were treated in Lysis Buffer (50mM Tris-HCl, 0.15 M NaCl, 1% Triton X-100, 1 mM EDTA), denatured in 2 x Laemmli Buffer (BioRad) at 95°C for 5 min, and separated by electrophoresis on Mini-PROTEAN TGX precast gels (BioRad). The total protein concentration in the cell lysates was determined using a BCA Protein Assay kit (Pierce). Gels after electrophoresis were blotted onto polyvinylidene fluoride (PVDF) membranes and blocked with 5 % BSA, then reacted with antibodies against phospho- or total STAT6, NF-κB p65, or Syk (all purchased from Cell Signaling Technology), and further probed with secondary anti-rabbit-IgG-HRP (Invitrogen) antibodies. Membranes were developed with the ECL Prime substrate (GE Healthcare).

### In vitro helper T cell differentiation and cytokine analysis

MACS sorted CD4⁺ T cells described above were further purified as CD4⁺CD25⁻CD62L⁺CD44⁻ naive T cells by FACS, and cells were seeded at a density of 3 x 10⁵ cells per 100 µl of RPMI culture medium described above in 96-well plates coated with 2 µg/ml anti-CD3 mAb and 2 µg/ml anti-CD28 mAb. Cytokines for effector cell differentiation were as follows: Th0, anti-IFN-γ (10 µg/ml; XMG1.2), and anti-IL-4 (10 µg/ml; 11B11); Th0, anti-IFN-γ (10 µg/ml; XMG1.2 (BD Pharmingen)), and anti-IL-4 (10 µg/ml; 11B11 (BD Pharmingen)); Th1, IL-12 (10 ng/ml (R&D Systems)), IL-2 (50 µg/ml (R&D Systems)), and anti-IL-4 (10 µg/ml; 11B11); Th17, TGF-β1 (1ng/ml), IL-6 (50 ng/ml (BioLegend)), IL-23 (50 ng/ml (R&D Systems)), anti-IFN-γ (10 µg/ml; XMG1.2), and anti-IL-4 (10 µg/ml; 11 B 11); induced Treg, TGF-β1 (5ng/ml) and anti-IL-4 (10 µg/ml; 11B11). The culture supernatants were harvested on day 5, and TGF-β3 levels were analyzed by ELISA as described above.

### In vitro CD4⁺Egr2⁺LAG3⁺ Treg differentiation by IL-27

*In vitro* stimulation of MACS purified naïve CD4⁺ T cells using the CD4⁺CD62L⁺T Cell Isolation Kit (Miltenyi Biotec) according to the manufacturer's protocol was performed in 24- or 96-well plates coated with 2 µg/ml of anti-CD3 mAb and 1 µg/ml of anti-CD28 mAb in the RPMI culture medium described above supplemented with 25 ng/ml IL-27 for 5 days. IL-27-treated naïve T cells were subsequently sorted by flow cytometry for CD4 expression and used for each assay. For determination of the TGF-β3 level in the culture supernatants by ELISA, IL-27-treated T cells were stimulated with PMA (50 ng/ml; Sigma) and ionomycin (µg/ml; Sigma) for the final 4 hours.

### Construction of the TGF-β3 expression plasmid vector

A full-length fragment of murine TGF-β3 was isolated from an OmicsLink™ Expression-Ready ORF-cloning vector (GeneCopoeia) containing a *Tgfb3* cDNA (NM_009368). The *Tgfb3* cDNA was subcloned using *EcoRI* sites into the pCAGGS vector (Non-Patent Document 50), which has the CAG (cytomegalovirus immediately-early enhancer/chicken β-actin hybrid) promoter, and was designated as pCAGGs-*Tgfb3*. Recombinant plasmids were then transformed into competent cells of *Escherichia coli* JM109 and purified on plasmid purification columns using EndFree Plasmid Giga Kit (Qiagen) according to the manufacturer's protocol. The purified plasmid DNA was diluted to 1 µg/µl with sterile PBS (pH 7.4) immediately before use.

### Intravenous injection of plasmid DNA

MRL/*lpr* mice were injected *i.v.* with 100 µg of plasmid DNA (pCAGGs-*Tgfb3* or control pCAGGS) in sterile PBS (pH 7.4) twice at an interval of 4 weeks. Proteinuria was assessed semiquantitatively at weekly intervals, and renal pathology was evaluated 6 weeks after the final administration as described above.

### Flow cytometric assessment of human PBMCs

All human samples were obtained under informed consent. The protocol for the human research project has been approved by the Ethics Committee of the University of Tokyo. PBMCs from healthy and SLE patients were isolated by Ficoll-Paque (Amersham Pharmacia Biotech) gradient. After the cells were washed, they were stained with the indicated mAbs for 20 minutes at 4°C. To prevent non-specific binding of mAbs, Human Fc Receptor Binding Inhibitor (eBioscience) was added before staining with labelled mAb. Dead cells were excluded by 7-AAD. The fluorescence-positive cells were analyzed by a Moflo XDP cell sorter. The five distinct subpopulations are as follows: (naïve T) CD4⁺CD25⁻CD45RA⁺CCR7⁺ cells; (CD25⁺ Treg) CD4⁺CD25⁺CD127^{dim}CD45RA⁻ cells; (LAG3⁺ Treg) CD4⁺CD25⁻CD45RA⁻LAG3⁺ cells; (Tfh) CD3⁺CD19⁻CD4⁺CD25⁻LAG3⁻CXCR5⁺CD45RA⁻ cells; (B cells) CD3⁻CD19⁺ cells.

### Quantitative real-time PCR expression analysis of human T cell subsets

Total RNA isolated from human naive T and CD25⁺ Treg, and LAG3⁺ Treg stimulated with plate-bound 5 µg/ml anti-CD3 mAb for 72 hours were analyzed for the mRNA expression of *EGR2, IL10, IFNG,* and *FOXP3,* as described above. The *TGFB3* mRNA expression was determined using unstimulated cells from each T cell subset.

### Quantification of human IL-10

For cytokines analysis, human naïve T, CD25⁺ Treg, and LAG3⁺ Treg were plated into 5 µg/ml anti-CD3 mAb/anti-CD28 mAb-coated 96-well flat-bottomed plates at 2 x 10⁴ cells per well in an RPMI-1640 medium supplemented with 10% FBS, 100 µg/ml L-glutamine, 100 U/ml penicillin, 100 µg/ml streptomycin, and 50 µM 2-mercaptoethanol. The culture supernatants were harvested on day 3, and the level of IL-10 in the culture supernatants was analyzed by ELISA using OptEIA Human IL-10 ELISA Kit II (BD Biosciences) according to the manufacturer's protocol.

### Human LAG3⁺ Treg suppression assays

The RPMI-1640 medium described above was used for co-culturing. 1 × 10⁵ of FACS-purified human B cells and 5 × 10⁴ of FACS-purified human T_{FH} cells were seeded in round-bottom 96-well plates with or without 1 × 10⁵ of FACS-purified human CD25⁺ Treg or LAG3⁺ Treg in the presence of 2 µg/ml of recombinant Staphylococcal enterotoxin B (Toxin Technology). The culture supernatants were harvested on day 12, and the levels of total IgG were analyzed by ELISA using Human IgG Quantitation Set kits (Bethyl Laboratories), according to the manufacturer's protocol.

### Statistical analysis

Statistical significance, normal distribution, and F test between groups were analyzed using GraphPad Prism version 5.03 (GraphPad Software, Inc.). Quantitative histology and proteinuria progression were analyzed with the Mann-Whitney *U*-test. All other statistical differences were determined using the two-tailed Student's *t*-test. If the variance was unequal, Welch's correction was applied to Student's t-test. Differences were considered statistically significant at *P* < 0.05 for all tests. All data in the figures are expressed as mean ± s.d. Sample size was estimated based on the numbers typically used in previous studies. No statistical method was used to predetermine sample size. No samples or animals were excluded from the analyses. Randomization of animals was not performed except for adoptive transfer studies in MRL/*lpr* mice. Animal studies were not performed in a blinded fashion except for histological analyses.

### [Results]

### Egr2-dependent suppression of humoral immunity by LAG3⁺ Treg

To clarify the role of Egr2 in T cells, the present inventors generated T cell-specific Egr2 conditional knockout (CKO) mice *(Egr2*^{*fl*/*fl*} CD4-*Cre*⁺)*.* Egr2 CKO mice showed significant increases in the proportion of CD4⁺CD25⁻CXCR5⁺PD-1⁺ T_{FH} and B220⁺GL-7⁺Fas⁺ follicular B cells (GCB) (Fig. 1a), and they demonstrated enhanced 4-hydroxy-3-nitrophenylacetyl (NP)-specific antibody production following a single immunization with NP-ovalbumin (NP-OVA) (Figs. 1b and 8a). Transfer of wild type (WT) LAG3⁺ Treg significantly suppressed the spontaneous differentiation of T_{FH} and GCB (Fig.1a) and inhibited excessive antibody production (Fig. 1b), indicating that LAG3⁺ Treg are able to suppress B cell responses *in vivo.* In an *in vitro* T cell/B cell co-culture system, anti-CD3 antibody-stimulated WT LAG3⁺ Treg more efficiently reduced the percentage of viable anti-IgM-stimulated B cells as well as total IgG production from anti-CD40 antibody/IL-4-stimulated B cells when compared to CD25⁺ Treg (Fig. 1c,d). To evaluate B cell responses *in vivo,* recombination-activating gene 1-deficient (Rag1KO) mice were transferred with B cells from WT mice and CD4⁺CD25⁻LAG3⁻ helper T cells (Th cells) from OVA-specific OT-II T cell receptor (TCR) transgenic mice, and then immunized with NP-OVA twice. Strikingly, co-transfer of WT LAG3⁺ Treg effectively suppressed NP-specific antibody responses and the development of T_{FH} and GCB (Figs. 1e,f and 8b). The enhanced GCB development and antibody production in Egr2CKO mice suggested a pivotal role for Egr2 in B cell regulation, and Egr2-deficient LAG3⁺ Treg (Fig. 8c) failed to suppress *in vivo* B cell antibody production and the development of T_{FH} and GCB (Fig. 1e,f). Thus, the expression of Egr2 on LAG3⁺ Treg is necessary for the suppression of B cell responses by LAG3⁺ Treg. In transgenic mice that expressed the green fluorescent protein (GFP) under the control of the Egr2 promoter (Egr2-GFP mice, Fig. 9a), the expression of GFP in CD4⁺ T cells correlated with the Egr2 protein expression (Fig. 9b). The importance of Egr2 was confirmed by the observation that CD4⁺CD25⁻Egr2⁻GFP⁺ cells from Egr2-GFP mice also exhibited B cell suppressive activity *in vivo,* similar to that of LAG3⁺ Treg (Fig. 9c). The present inventors next determined whether the suppression of antibody production via LAG3⁺ Treg is induced not only under lymphopenic conditions, such as in RaglKO mice, but also under more physiological non-lymphopenic conditions. Eα peptide-specific TCR transgenic TEa mice were adoptively transferred with WT B cells and OT-II Th cells and subsequently immunized with NP-OVA once. Co-transferring WT LAG3⁺ Treg effectively suppressed NP-specific antibody production in non-lymphopenic TEa mice (Figs. 1g and 8d).

### LAG3⁺Treg suppress a lupus-like disease in Fas-mutated MRL/lpr mice

The present inventors investigated whether LAG3⁺ Treg were able to inhibit disease progression in lupus-prone MRL*-Fas*^{*lpr*/*lpr*} (MRL/*lpr*) mice with a *Fas* mutation (Non-Patent Document 17). MRL/*lpr* mice were adoptively transferred with various T cell subsets from MRL*-Fas*^{+/+} (MRL/+) mice not having a Fas mutation. LAG3⁺ Treg but not CD25⁺ Treg significantly delayed proteinuria progression (Fig. 2a). Furthermore, the three-time transfer of LAG3⁺ Treg almost completely suppressed proteinuria progression. Increases in anti-ds-DNA antibody titers and glomerular pathology scores were also inhibited by the single transfer of LAG3⁺ Treg (Figs. 2b-d and 10a). In contrast, consistent with previous reports (Non-Patent Document 18), the three-time transfer of CD25⁺ Treg from MRL/+ mice to MRL/*lpr* mice did not alter the disease progression (Fig. 10b-d). Furthermore, adoptive transfer of LAG3⁺ Treg from MRL/*lpr* mice had no therapeutic benefit in MRL/*lpr* mice (Fig. 10b-d). The three-time injection of MRL/+ LAG3⁺ Treg also ameliorated lupus pathologies in MRL/*lpr* mice after the onset of overt proteinuria (Fig. 10e,f).

The therapeutic effect of LAG3⁺ Treg from MRL/+ in Fas-mutated MRL/*lpr* mice suggested that Fas contributes to the suppressive ability of LAG3⁺ Treg. The present inventors examined the correlation between suppressive ability on humoral immunity of LAG3⁺ Treg and Fas expression in B6 background mice. Adding anti-FasL blocking antibody abrogated LAG3⁺ Treg-mediated antibody suppression both *in vitro* (Figs. 2e and 8e) and *in vivo* (Fig. 2f-i). LAG3⁺ Treg from Fas-deficient B6/*lpr* mice, but notLAG3⁺ Treg from FasL-deficient B6/*gld* mice, failed to suppress antibody production (Fig. 2f-i). Therefore, Fas, but not FasL, on LAG3⁺ Treg is required to suppress B cells. Fas expression on CD4⁺ T cells was independent of Egr2 because activated CD4⁺ T cells from WT and Egr2CKO mice expressed similar levels of Fas on LAG3⁺ cells (Fig. 11).

### Involvement of TGF-β3 produced by LAG3⁺ Treg in the control of humoral immunity

We next examined whether B cell suppression by LAG3⁺ Treg is mediated by IL-10 or TGF-β family members. As described above, LAG3 is considered to be one of the specific cell-surface markers for Tr1 cells (Non-Patent Document 16). The present inventors previously reported that LAG3⁺ Treg produce large amounts of IL-10 (Non-Patent Document 14), and Egr2 mediates IL-27-induced IL-10 production in CD4⁺ T cells through B lymphocyte induced maturation protein-1 (Blimp-1) (coded by *Prdm1* gene) (Non-Patent Document 19). As expected, IL-10 expression levels were significantly reduced in LAG3⁺ Treg from T cell specific Prdm1CKO mice (*Prdm1*^{*fl*/*fl*} CD4*-Cre*⁺) compared to WT mice (Fig. 12a). LAG3⁺ Treg derived from Prdm1CKO and IL-10-deficient (IL-10KO) mice effectively suppressed *in vivo* NP-specific antibody responses (Fig. 12b), indicating that IL-10 may not be critical for B cell suppression by LAG3⁺ Treg. Microarray analysis (Non-Patent Document 14) and quantitative real-time PCR of LAG3⁺ Treg revealed a significant increase in TGF-β3 expression, but not TGF-β1 or 2 (Fig. 3a,b). T cell receptor (TCR) stimulation induced the production of a large amount of TGF-β3, but not TGF-β1 or 2, in the culture supernatants of LAG3⁺ Treg (Fig. 3c-e). In contrast, CD25⁺ Treg only produced small amounts of TGF-β1 under these conditions. TGF-β markedly suppressed anti-IgM-stimulated B cell proliferation and CD40 expression (Fig. 3f), strongly induced B cell death (Fig. 3g), and suppressed total IgG production (Fig. 3h). TGF-β produced similar effects as TGF-β1 and 2 (Fig. 13a,d), in accordance with previous findings that TGF-β1 strongly suppresses B cell functions (Non-Patent Document 20). Regarding signal transduction, the addition of TGF-β3 significantly reduced the phosphorylation of signal transducer and activator of transcription (STAT) 6, Syk, and NF-κB p65 in activated B cells (Fig. 3i-k). IL-4 produced by helper T cells enhances the proliferation and survival of B cells, while promoting immunoglobulin secretion and isotype switching via the activation of STAT6 (Non-Patent Document 21). Activation of the tyrosine kinase Syk is critical for the cell signaling in response to B cell receptor (BCR) stimulation (Non-Patent Document 22). Activation of CD40, which is required for specific antibody production by antigen-stimulated B cells, induces phosphorylation of NF-κB p65 (Non-Patent Document 23). Therefore, TGF-β3 inhibits several important pathways for B cell functions. TGF-β3 production is not limited to LAG3⁺ Treg, because TGF-β3 is also produced by differentiating Th17 cells in an IL-23-dependent manner (Non-Patent Document 24). The present inventors found that Th1 cells produced TGF-β3 in addition to Th17 cells (Fig. 31). However, LAG3⁺ Treg produced significantly greater amounts of TGF-β3 than Th1 and Th17 cells.

Administration of a TGF-β3 blocking antibody cancelled the LAG3⁺ Treg co-transfer-mediated suppression of antibody production and the development of T_{FH} and GCB in RaglKO mice transferred with WT B cells and WT OT-II Th cells and immunized twice with OVA-NP (Fig. 4a,b). The TGF-β3 blockade also abrogated the therapeutic effects of MRL/+ LAG3⁺ Treg in MRL/*lpr* mice (Fig. 4c,d), indicating a critical role for TGF-β3. Intriguingly, TGF-β3 production by LAG3⁺ Treg from Egr2-deficient mice and LAG3⁺ Treg from Fas-mutated B6/*lpr* was markedly reduced (Fig. 4e). Therefore, Egr2 and Fas are required for the production of TGF-β3 and the B cell suppressive activity of LAG3⁺ Treg. The importance of TGF-β3 for the control of lupus pathology in MRL/*lpr* mice has been verified by the observation that administration of a TGF-β3-expressing plasmid by naked DNA method significantly improved proteinuria progression and renal pathology (Fig. 4f,g).

### Necessity of PD-1 expression on B cells for LAG3⁺ Treg-mediated suppression

Variants of the programmed cell death-1 (*PD-1*) gene have been associated with SLE susceptibility (Non-Patent Document 1). PD-1 provides negative co-stimulatory signals to both T cells and B cells (Non-Patent Documents 25 and 26) and PD-1-deficient (PD-1KO) mice develop a lupus-like disease (Non-Patent Document 27). Furthermore, PD-1 and LAG3 synergistically regulate autoimmunity and tumor immunity (Non-Patent Documents 28 and 29). To examine the potential cooperation between rTGF-β3 and PD-1, the present inventors added TGF-β3 to anti-IgM antibody-stimulated B cells from B6, PD-1KO, Fas-deficient B6/*lpr*, and FasL-deficient B6/*gld* mice. PD-1KO mice-derived B cells, but not B6/*lpr* mice-derived or B6/*gld* mice-derived B cells, were resistant to the TGF-β3-induced inhibition of cell division (Fig. 5a). In accordance with a previous report that phosphorylated STAT6 is known to induce the expression of anti-apoptotic Bcl-xL in B cell lines (Non-Patent Document 30), TGF-β3 suppressed the expression of Bcl-xL and Bcl-2a1 in activated WT mice-derived B cells, but not in PD-1KO B cells (Fig. 5b). The cooperative suppression of B cells by TGF-β3 and PD-1 underscores the importance of PD-1 expression on B cells for LAG3⁺ Treg-mediated suppression. The addition of an anti-PD-L1 blocking antibody reversed the LAG3⁺ Treg-mediated suppression of antibody production *in vitro* (Fig. 5c). Anti-NP antibody production was not suppressed by the co-transfer of WT LAG3⁺ Treg in Rag1KO mice transferred with PD-1KO B cells and WT OT-II Th cells and immunized twice with OVA-NP; however, Rag1KO mice transferred with WT B cells and PD-1KO OT-II Th cells were suppressed by LAG3⁺ Treg (Fig. 5d). These results confirm that PD-1 expression on B cells is required for LAG3⁺ Treg-mediated B cell suppression. CD4⁺CD25⁻Egr2⁺ T cells co-expressed both PD-L1, the ligand for PD-1, and LAG3 (Fig. 14a,b). However, because PD-L1 and PD-L2 are expressed on various cell types including GCB (Non-Patent Document 31), PD-L1 on LAG3⁺ Treg may not be the only ligand for PD-1 on B cells.

### IL-27-mediated induction of TGF-β3-producing Egr2⁺ T cells with B cell response-suppressing function

IL-27 is a member of the IL-12/IL-23 heterodimeric family of cytokines produced by antigen presenting cells (APCs). IL-27 has been identified as a differentiation factor for IL-10 producing Tr1 cells (Non-Patent Document 32). The present inventors have previously reported that IL-27 induces Egr2 expression in CD4⁺ T cells and Egr2 is required for the Blimp-1-mediated IL-10 production (Non-Patent Document 19). IL-27 treatment induced not only Egr2 and LAG3 (Fig. 6a) but also production of TGF-β3 mRNA and TGF-β3 protein (Fig. 6b,c). Egr2-deficient CD4⁺ T cells exhibited a substantial reduction in IL-27-induced production of TGF-β3 (Fig.6c), confirming the importance of Egr2 for the induction of TGF-β3. The activation of specific STAT proteins in CD4⁺ T cells is associated with the differentiation of helper T cell lineages. Although IL-27-mediated IL-10 induction requires both STAT1 and STAT3 (Non-Patent Document 32), we previously found that IL-27-mediated induction of Egr2 is dependent on STAT3 (Non-Patent Document 19). IL-27-mediated TGF-β3 induction was impaired in STAT3-deficient CD4⁺ T cells, not STAT1-deficient CD4⁺ T cells (Fig.6c), demonstrating similarity between Egr2 and TGF-β3 inductions in STAT3 dependency. Moreover, IL-27-treated CD4⁺ T cells significantly suppressed B cell antibody production (Fig.6d). In contrast, Egr2-deficient CD4⁺ T cells treated with IL-27 failed to suppress antibody production by B cells. Thus, IL-27 induced TGF-β3-producing cells exhibit suppressive activity on humoral immunity in an Egr2-dependent manner.

### Mechanism of suppression of antibody production by human LAG3⁺ Treg, and correlation between human LAG3⁺Treg and systemic lupus erythematosus

The present inventors identified CD4⁺CD25⁻CD45RA⁻LAG3⁺ T cells in CD4⁺ T cells from peripheral blood mononuclear cells (PBMCs) of healthy donors (Fig.7a). Similar to murine LAG3⁺ Treg, human CD4⁺CD25⁻CD45RA⁻LAG3⁺ T cells expressed *EGR2, IL10,* and *IFNG* (Fig.7b) and produced significant amounts of IL-10 in response to TCR stimulation (Fig.7c). The regulatory activity of human CD4⁺CD25⁻CD45RA⁻LAG3⁺ T cells was confirmed by the observation that they more efficiently suppressed antibody production when co-cultured with B cells and T_{FH} cells compared to CD4⁺CD25⁻CD127^{low}CD45RA⁻ activated Treg (Non-Patent Document 33) (Fig.7d). Human CD4⁺CD25⁻CD45RA⁻LAG3⁺ T cells expressed high levels of TGF-β3 after culturing (Fig.7e), suggesting that they suppress B cells through a mechanism identical to murine LAG3⁺ Treg. Therefore, the present inventors consider the CD4⁺CD25⁻CD45RA⁻LAG3⁺ T cell population as the human counterpart to murine LAG3⁺ Treg. The present inventors next assessed whether LAG3⁺ Treg might be reduced in human systemic autoimmune diseases. The percentages of blood LAG3⁺ Treg were significantly lower in the peripheral blood of SLE patients compared to those in healthy donors (Fig. 7f). These findings suggest that LAG3 expression could be used for monitoring the T cell population with antibody-suppressing capacity in SLE patients.

### Comparison of therapeutic effects of TGF-β3 and TGF-β1 in SLE model mice

It is understood that generally TGF-β3 and TGF-β1 show similar biological activities, at least *in vitro, via* common signaling molecules. However *in vivo,* mainly through results from knockout mice, the two have been reported to have different phenotypes, and this difference is caused by the timing and location of expression; alternatively, since TGF-β1 KO mice, which are inherently lethal, that have a TGF-β3 knock-in are rescued but also show different phenotypes, there has also been suggested the possibility that the two show different biological activities in *vivo* (Non-Patent Document 51).

On the other hand, from reports on immune systems, TGF-β3 has been reported to induce pathogenic T cells (Thl7 cells) and worsen pathological conditions in experimental autoimmune encephalomyelitis (EAE) models, a report which is opposite from the current experimental results (Non-Patent Document 52). This document shows that this action is absent in TGF-β1. Similarly, involvement of TGF-β3 in aggravation of autoimmune diseases has been reported in TRIM28 KO mice as well (Patent Document 52). These mice are short lived due to spontaneous autoimmune diseases and show worsened EAE, and TGF-β3 derepression is considered to be involved therein.

As such, existing reports suggested that TGF-β3 and TGF-β1 have different activities *in vivo.* Accordingly, to comparatively investigate the immunosuppressive actions of TGF-β3 observed this time with respect to the actions of TGF-β1, the present inventors examined the effects of expression of the two genes on SLE-like conditions by expressing genes encoding the full-length TGF-β1 and TGF-β3 in MRL/*lpr* mice, which are spontaneous models of SLE. Full-length TGF-β1 and TGF-β3 cloned into pCAGGS plasmids and a control pCAGGS plasmid were each administered at 100 µg to MRL/*lpr* mice through the caudal vein. Each plasmid was administered twice, *i.e.,* at 11 weeks and 15 weeks of age, and subsequently, various pathological parameters were measured.

As a result, the TGF-β3 expression plasmid significantly suppressed the spleen weight (at 21 weeks of age) and the increase of proteinuria over time in comparison to the control plasmid, and these effects tended to be stronger than those of the TGF-β1 expression plasmid (Fig. 15a, b). Furthermore, TGF-β3 expression plasmid was found to inhibit deposition of IgG in the kidneys, and in addition, glomerular lesions assessed using cell infiltration and proliferation, crescent formation, cell death, and such as indicators were found to be significantly reduced as compared with the control plasmid and TGF-β1 expression plasmid (Fig. 16a, b). Furthermore, while the TGF-β1 expression plasmid significantly increased the percentage of CD4/CXCR5/PD-1-positive CD25-negative follicular helper T cells (TFH) present in germinal centers of secondary lymphoid tissues including lymph nodes and the spleen, this was significantly suppressed by the TGF-β3 expression plasmid (Fig. 17a, b).

The above-mentioned results suggested that the difference in therapeutic effects of TGF-β1 and TGF-β3 in MRL/*lpr* mouse models may depend on the presence or absence of the ability to suppress follicular helper T cells that are important for immune responses involving antibody production. Thus, TGF-β3 may be useful in the treatment of autoimmune diseases such as SLE through suppression of follicular helper T cells, which is an action not found in TGF-β1.

### [Discussion]

The results of the present study demonstrated that LAG3⁺ Treg suppress the development of GCB and T_{FH}, antibody production, and disease progression in MRL/*lpr* mice showing lupus-like pathologies. TGF-β3, which is produced by LAG3⁺ Treg in Egr2-dependent and Fas-dependent manners, plays a critical role in suppressing humoral immunity. As LAG3⁺ Treg also produce much higher levels of IL-10 than CD25⁺ Treg (Non-Patent Document 44), LAG3⁺ Treg are potent producers of regulatory cytokines. The pro-inflammatory role of TGF-β3 was previously demonstrated by the observation that TGF-β3 efficiently induces pathogenic Th17 cells (Non-Patent Documents 24 and 34). The results of the present inventors have revealed a previously unrecognized role for TGF-β3 in the control of autoimmunity. TGF-β1 also exerts both pro-inflammatory and anti-inflammatory effects (Non-Patent Documents 35-37). In particular, TGF-β1 induces B cell apoptosis and reduces immunoglobulin production from activated human tonsil B cells (Non-Patent Documents 38 and 39). CD25⁺ Treg and Th3 regulatory cells (Non-Patent Documents 40 and 41) are potent sources of TGF-β1, and CD25⁺ Treg have been shown to suppress B cell immunoglobulin synthesis through TGF-β1 (Non-Patent Document 42). However, the amount of TGF-β1 produced by CD4⁺ T cells including CD25⁺ Treg is relatively limited (Fig. 3e), and it has been difficult to define the sources of TGF-β1 that are relevant to immune suppression. Although it was demonstrated in a number of systems that TGF-β1 and TGF-β3 display clear isoform-specific biological activities, TGF-β1 and TGF-β3 showed comparable suppressive activities on B cell responses (Fig. 13a,b). In terms of helper T cell development, TGF-β3 is autonomously produced by Th17 cells during the development of pathogenic Th17 cells (Non-Patent Document 24). In the setting of the present inventors, not only Th17 cells but also Th1 cells produced significant amounts of TGF-β3; however, LAG3⁺ Treg produced greater amounts of TGF-β3 compared to Th1 and Th17 cells (Fig. 31). Therefore, the large amount of TGF-β3 produced by LAG3⁺ Treg plays a significant role in the generation and maintenance of immune tolerance.

The present inventors identified two molecules, Fas and Egr2, which are required for TGF-β3 secretion in LAG3⁺ Treg. Egr2 deficiency in T cells and B cells results in a lupus-like syndrome, and Egr2 directly activates p21^{cipl} expression in CD44^{high} T cells and is involved in the control of Th1 and Th17 differentiation. The fact that Egr2 blocks the function of BATF, an AP-1 inhibitor required for the differentiation of Th17 cells, indicates that Egr2 is an intrinsic regulator of effector T cells (Non-Patent Document 12). The present inventors showed here that Egr2 in T cells is important for the control of the development of T_{FH} and GCB (Fig.1). Extrinsic functions of Egr2 for regulating humoral immunity were confirmed by the observation of the present inventors that transfer of Egr2-expressing LAG3⁺ Treg suppressed the excessive expansion of T_{FH} and GCB as well as antibody production in Egr2CKO mice (Fig. 1). Fas controls T cell and B cell expansion by triggering apoptosis. As administration of TGF-β3 ameliorated autoimmune disease pathology in MRL/*lpr* mice, the impaired production of TGF-β3 by LAG3⁺ Treg may be a cause of the disease (Fig. 4f,g).

IL-27, a differentiation factor for IL-10-producing Tr1 cells (Non-Patent Document 32), induces CD4⁺Egr2⁺LAG3⁺ T cells (Non-Patent Document 19). However, the role of Tr1 cells in the regulation of B cells is not clear because CD46-induced Tr1 cells are more potent at enhancing immunoglobulin production compared with conventional T cells (Non-Patent Document 44). In LAG3⁺Treg, IL-10 did not directly contribute to the control of B cell response (Fig. 12). Nevertheless, the linkage between IL-27 and the control of antibody production was suggested by the observation that overexpression of the IL-27 receptor, WSX-1, protects MRL/*lpr* mice from the development of autoimmune disease. It is notable that whereas STAT1 and STAT3 are required for the induction of IL-10 by IL-27, the activity of IL-27 to promote Egr2 and TGF-β3 is STAT3-dependent (Fig.6c). As STAT3-activating IL-6 also induces TGF-β3 production (Non-Patent Document 24), STAT3 may play key roles for TGF-β3 induction in CD4⁺ T cells.

### [Applications to TGF-β3 functional molecules]

Generation of novel functional molecules using TGF-β3 was investigated by the present inventors based on the findings on suppression of B cell activation by TGF-β3. A conceivable configuration of the functional molecule is, for example, one in which TGF-β3 is linked with an antibody Fc region through amino acids and the antibody further contains a variable region that recognizes B cells. TGF-β3 can be linked with the antibody or antibody fragment through a linkage using the third TB domain (TGFbeta binding domain) of TBP (latent TGFbeta-binding protein)-1, 3, or 4; a linkage using an enzyme; or a linkage using a chemical modification reaction.

Other means include configuration of a multi specific antibody where one variable region recognizes TGF-β3 and the other variable region recognizes B cells. Another configuration is that of a fusion protein in which TGF-β3 is linked with an antibody Fc fragment, and TGF-β3 is further linked with a variable region fragment that recognizes B cells.

The B cells are desirably autoreactive B cells, and B cell recognition by the variable region may be accomplished by B-cell surface markers such as CD 19, CD20, CD40, CD22, IL21R, BAFF-R, BCMA, TACI, CD27, and CD138.

To provide biologically active proteins such as TGF-β3 as therapeutic agents, one may consider working the proteins stably in human plasma. As an embodiment for allowing stable action in plasma, the proteins may be linked with a protein that has long half-life in plasma such as an antibody or serum albumin. It may also be chemically linked to polyethylene glycol (PEG) or polysaccharides, or modified by glycosylation. There are also recent reports of techniques of mimicking PEG by linking a long artificial polypeptide sequence (Non-Patent Document 53).

These functional molecules stably transport TGF-β3 to B cells and effectively suppress the targeted B cell activity, and can also be applied as therapeutic agents for autoimmune diseases. Regarding autoimmune diseases, in addition to SLE to which TGF-β3 was confirmed to have improving effects, similar improving effects are expected for diseases that involve IgG production or T_{FH} increase, such as, pemphigus, multiple sclerosis, neuromyelitis optica (NMO), ANCA-associated vasculitis, rheumatoid arthritis, organ transplant rejection, Sjogren's syndrome, juvenile dermatomyositis, polymositis / dermatomyositis, systemic sclerosis, myasthenia gravis, and autoimmune thyroid disease including Graves' disease or Hashimoto's thyroiditis.

### Generation of latent TGFβ3 and TGFβ3-modified antibody molecules Gene construction

The mouse TGFβ3 gene was prepared by using positions 24 to 410 of an amino acid sequence registered on database (Uniprot No. P17125); and for expression as latent TGFβ3, a mouse Ig kappa chain signal sequence was added to the N-terminal side, and to facilitate simple purification, a FLAG tag was fused immediately after the signal sequence. Furthermore, to improve the expression level of latent TGFβ3, Cys at position 25 in the database-registered amino acid sequence (Uniprot No. P 17125) was substituted with Ser according to the information described in the publication, Molecular Therapy (2010) 18:12, 2104-2111. The genetic sequence inserted in the OmicsLink™ Expression-Ready ORF cloning vector (GeneCopoeia) was used as the wild-type TGFβ3 gene. The amino acid sequence of latent TGFβ3, excluding the signal sequence, is shown in SEQ ID NO: 11.

The TGFβ3-modified antibody molecule has a molecular shape that possesses a mouse CD19-recognizing antibody on one arm, and was prepared by attaching to its C terminus a TB3 domain (the third TB domain (TGFbeta binding domain) of TBP (latent TGFbeta-binding protein)-1, 3, and 4) for linking TGFβ3. For comparison, antibodies that do not have the TB3 domain for linking TGFβ3 were also prepared. These modified antibody molecules have a structure in which one Fab of an anti-CD19 antibody is linked to an Fc.

The antibody variable region gene of the 1D3 antibody which recognizes mouse CD 19 was cloned according to standard methods from rat hybridoma 1D3 (ATCC® HB-305™) commercially available from ATCC. The cloned L-chain variable region was linked with the mouse kappa chain constant region. The cloned H-chain variable region was linked with the modified constant region sequence indicated below. For the H-chain constant region, firstly, to promote heterologous formation of the Fc region, the CH3 domain D399R is introduced into the Fc region mF18 which has been introduced with amino acid mutations to lose the binding to the Fc receptors (i.e., carrying amino acid mutations where Pro at position 235 according to EU numbering is substituted with Lys and Ser at position 239 is substituted with Lys). Furthermore, the sequences of V5 tag for use in detection and purification and the third TB domain (TGFbeta binding domain) (TB3) of the mouse LTBP1 protein (SEQ ID NO: 9) for the conjugation of latent TGFβ3 was linked to the C terminal side. The above-mentioned mutations for not binding to Fc receptors and K409E as mutation to promote heterologous formation were also introduced into the H chain constant region that only has the Fc region (SEQ ID NO: 10). Details on the mutations for not binding to Fc receptors are described in published patent WO 2012-132067, and details on the mutations to promote heterologous formation are described in published patent WO2015-046467.

These genes were cloned into a pBEF-OriP vector for transient expression in mammalian cells (Wako). Transfection-grade samples were prepared from the obtained plasmids using a plasmid preparation kit (Macherey-Nagel, NucleoBond Xtra Maxi), and they were used for the expression experiments. The gene names and protein names of the expressed proteins are shown in Table 1.

**[Table 1]**

| Used plasmid | | |
|---|---|---|
| | Gene name | Expressed protein name |
| Latent TGFbeta3 | ssIgK-FLAG-TGFbeta3(C25S) | Latent TGFbeta3 |
| | | |
| Modified | HC(1D3V_{H}-mF18)-V5-TB3(D399R) | 1D3-mF18 |
| antibody | LC(1D3V_{L}-mk0) | |
| molecule | nonV_{H}-mF18Fc(K409E) | |
| | | |
| TGFbeta3 | HC(1D3V_{H}-mF18)-V5-TB3(D399R) | 1D3-mF18-latent TGFbeta3 |
| modified | LC(1D3V_{L}-mk0) | |
| antibody | nonV_{H}-mF18Fc(K409E) | |
| molecule | WT-TGFbeta3 | |

### Expression

Expi293-F cells (Life technologies, A14527) and a transfection kit (ExpiFectamine293 transfection kit) therefor were used to express these proteins. Expi293-F cells were cultured using the Expi293 medium (Life technologies) in a CO₂ incubator at 37°C by shaking at 135 rpm. Plasmids were introduced according to the protocol by Life technologies, and in addition to TGFβ3 and antibody plasmids, pBEF-EBNA1 was added. The enhancer solution included in the kit was not used. As a result of examination, the culturing period was set to 5 or 7 days. After completion of culturing, the culture was centrifuged, and the supernatant was filtered through a 0.45-µm filter. The obtained culture supernatant was frozen in liquid nitrogen, and then stored in a -80°C freezer.

### Examination of latent TGFβ3 expression

Latent TGFβ3 was expressed using a ssIgK-FLAG-TGFbeta3(C25S) construct, and by co-expressing 30 µg of the plasmid and 2.4 µg of pBEF-EBNA1 per 30 mL of culture. Since maximum was reached with a culture period of seven days, the subsequent experiments were carried out by culturing for seven days. The percentage of the Pro-form increased when the Enhancer 1 and 2 solutions included in the transfection kit were added; therefore, they were not used.

### Purification of latent-TGFβ3

AKTA avant (GE Healthcare) was used to apply 200 mL of the culture supernatant onto a column packed with 2 mL of FLAG-M2 resin (anti-FLAG M2 agarose affinity gel #A2220 from Sigma) which had been equilibrated with a 50 mM HEPES /150 mM NaCl/1mM EDTA solution at pH 7.8. The experiment was performed in a cold room. After application at a flow rate of 1 ml/min, the column was washed using an equilibration buffer. Elution was performed using an equilibration buffer containing 0.1 mg/ml FLAG peptide (#F3290 from Sigma). The fractions containing latent-TGFβ3 were combined and dialyzed against a 20 mM Tris-HCl, pH8.0 /1mM EDTA solution. The dialyzed fraction was adsorbed onto an ENrich-Q column (10 x 100 mm #780-0003 from BioRad), and then eluted with a NaCl solution using a concentration gradient of 0-300 mM / 45 CV. When the separation from the Pro-form was not satisfactory, further purification was undertaken using a RESOURCE-Q 1ml column (#17-1177-01 from GE healthcare). After separation by an ion-exchange column, the sample was concentrated using an ultrafiltration membrane, and this was purified through a Superdex 200 increase 10/300 gel filtration column (#28-9909-44 from GE healthcare). The gel filtration chromatogram of latent-TGFβ3 is shown in Fig. 18. This gel filtration fraction was concentrated and used in subsequent experiments.

The fractionated fractions were analyzed by SDS-PAGE. 10-20% gradient gels or 12.5% gels from DRC were used, and reducing or non-reducing reagents from Nacalai were used for the sample buffer. A sample buffer was added at one-third of the volume of each of the fractions, and then this was subjected to heat treatment at 95°C for five minutes to prepare samples for electrophoresis. Protein marker (PrecisionPlus protein All blue standards #161-0373) from BioRad was used as the molecular weight marker. Images of electrophoresis of the gel filtration fractions are shown in Fig. 19.

### Examination of expression of the TGFβ3-modified antibody molecule

The TGFβ3-modified antibody molecule (1D3-mF18-latent TGFβ3) and the modified antibody molecule (1D3-mF18) were expressed using the plasmids inserted with the genes shown in Table 1. Both molecules used the same plasmids for the antibody H chain and L chain, and expression of the TGFβ3-modified antibody molecule involved co-expression of the TGFβ3 expression plasmid for conjugation to the C terminal side of the antibody. Expression conditions were examined to optimize the expression of the conjugate. Table 2 shows the amount of plasmids used for the expression. The ratio of the amount of TGFβ3 plasmid to the antibody was examined by increasing it from one equivalent to two-fold, and up to five-fold, and as a result, the conjugate was found to be maximized at two-fold, and the binding ratio was found to be maximized at 5-fold the amount of the antibody (Fig. 20). In later examinations, experiments were performed at this plasmid ratio of two-fold or five-fold the amount of the antibody. Since the amount of the conjugate reached a maximum after a culture period of seven days similarly with the latent form, culturing was performed for seven days.

**[Table 2]**

| Amount of plasmid used (amount per 30 ml of culture) | | | | | |
|---|---|---|---|---|---|
| | TGFbeta3 | H chain | L chain | nonV_{H}-Fc | EBNA1 |
| 1D3-mF18 | - | 15 µg | 15 µg | - | 2.4 µg |
| 1D3-mF18 -latent TGFbeta3 | 7.6 µg | 7.6 µg | 7.6 µg | 7.6 µg | 2.4 µg |
| 1D3-mF18 -latent TGFbeta3(2x) | 12 µg | 6 µg | 6 µg | 6 µg | 2.4 µg |
| 1D3-mF18 -latent TGFbeta3(5x) | 19 µg | 3.8 µg | 3.8 µg | 3.8 µg | 2.4 µg |

### Purification of the TGFβ3-modified antibody molecule

A single-arm antibody and an ordinary antibody (2 arms) were expressed with a TGFβ3 to antibody plasmid ratio of 2:1 or 5:1. For every 100 mL of the obtained culture supernatant, 1 mL of antiV5-tag resin (V5-tagged protein purification gel #3316 from MBL) equilibrated with PBS was added, and the antibodies were adsorbed onto the resin by shaking at 4°C for 15 hours. This resin was packed into a 10-mL centrifuge column (PIERCE), and then washed with PBS using ten-times the column volume. Elution was performed with one column volume of 2 mg/mL of the V5 peptide in PBS (V5-tag peptide #3315-205 from MBL) for 30 minutes at room temperature. This was repeated five times to obtain the eluate. This eluate was concentrated using an ultrafiltration membrane (Amicon Ultra MWCO10K, #UFC901024 or #UFC801024 from Millipore), and then applied to a gel filtration column.

The gel filtration purification was carried out on an AKTA avant purification system (GE Healthcare), using a Superdex 200 increase 10/300 (#28-9909-44 from GE healthcare) column. PBS was used for the equilibration buffer. A gel filtration chromatogram of the single arm 1D3-mF18-latent TGFβ3 conjugate is shown in Fig. 21.

As a result of electrophoresis (Fig. 22), Peak 1 which eluted first was the molecule with conjugated latent TGFβ3, and therefore, this was collected and used.

### Confirmation of the biological activities of latent TGFβ3 and the TGFβ3-modified antibody molecule

The biological activities of the prepared latent TGFβ3 and the TGFβ3-modified antibody molecules (single arm 1D3-mF18-latent TGFβ3 conjugate or single arm 1D3-mF18 without conjugation of TGFβ3) were confirmed using HEK-Blue_TGFb cells (hkb-tgfb, InvivoGen). Regarding the evaluation method for biological activity of mature TGFβ3 released by acid treatment or protease treatment, input of the Smad signal was confirmed by evaluating the secretory alkaline phosphatase (SEAP) activity.

Samples prepared by adding 5 µg/mL of the Plasmin (P1867, Sigma) protease to latent TGFβ3 or TGFβ3-modified antibody molecule, and mature TGFβ3 recombinant protein (243-B3-010/CF, R&D systems) dissolved in 4 mM HCl, which was used as the standard, were dispensed into a flat-bottom 96-well plate, the HEK-Blue_TGFb cell suspension solution was added at 5 x 10⁴ cells/well, and the cells were cultured overnight (20 hours) at 37°C in a CO₂ incubator. On the following day, 180 µL of the QUANTI-Blue (rep-qb1, InvivoGen) chromogenic substrate was added to 20 µL of the cell culture supernatant, and after incubating for one hour at 37°C in a CO₂ incubator, the secretory alkaline phosphatase (SEAP) activity was measured by VersaMax spectrometer (Molecular devices) at OD 655 nm.

While the EC50 of the mature TGFβ3 recombinant protein was approximately 0.2 ng/mL, the plasmin protease-treated latent TGFβ3 and the TGFβ3-modified antibody molecule (1D3-mF18-latent TGFβ3) showed biological activities with an EC50 value of approximately 10 ng/mL or so. On the other hand, the antibody molecule with no conjugated TGFβ3 (1D3-mF18) did not show any biological activity even when treated with the plasmin protease (Fig. 23).

### Binding specificity of the TGFβ3-modified molecule to B cells

Binding specificity to B-cell of the TGFβ3-modified antibody molecule was examined by FACS analysis. Spleen was isolated from 10-14-week-old male C57BL/6N mice purchased from Japan Charles River. Splenocytes were prepared and resuspended in FACS buffer, and were reacted with the TGFβ3-modified antibody molecule and fluorescence-labeled antibodies against markers for T cells, B cells, NK cells, monocytes, dendritic cells, and such. The following cell surface markers were used as indicators: B220 (BUV395, 563793, BD) and CD138 (APC, 132506, Biolegend) for specificity to B cells; CD3e (BUV737, 564618, BD), CD4 (PE-CF594, 562285, BD), CD8 (PE-Cy7, 552877, BD), and CD25 (APC, 102012, Biolegend) for T cells; CD11b (PE, 553311, BD), CD11c (APC, 550261, BD), and Gr-1 (FITC, 553127, BD) for lymphoid DC cells, plasmacytoid DC cells, myeloid DC cells, and macrophages; and CD11b (PE, 553311, BD) and CD49b (DX5) (BV421, 563063, BD) for NK cells; and fluorescence-labeled antibodies that recognize the respective antigens were combined. Subsequently, an antibody that recognizes the V5 tag conjugated to TGFβ3-modified antibody molecule (rabbit anti-V5 Ab, V8137, Sigma) and BV421-conjugated donkey anti-rabbit IgG Ab (406410, Biolegend) were individually reacted at 4°C for 30 minutes, washed twice using a FACS buffer, and then, those cells were fractionated using a FACS analyzer (LSRFortessaX-20, BD) to confirm the cell specificity of the TGFβ3-modified antibody molecule.

As a result of FACS analysis, since the splenocytes were not from disease models, staining of CD138-positive plasma cells could not be evaluated; however, binding specificity to B220-expressing B cells could be confirmed (Fig. 24).

### Smad signal input effects of the TGFβ3-modified antibody molecule

The biological activity to actually input Smad signaling of the TGFβ3-modified antibody molecule which was confirmed to specifically bind to B cells was evaluated by detection of phosphorylated Smad2/3. Spleen was isolated from 10-14-week-old male C57BL/6N mice purchased from Japan Charles River. Splenocytes were prepared and resuspended in PBS containing 0.5% BSA; latent TGFβ3, the TGFβ3-modified antibody molecule, and 5 µg/mL of Plasmin protease were added and incubated for 30 minutes at 37°C in a CO₂ incubator; then the cells were fixed with BD Phosflow™ Lyse/Fix buffer (558049, BD); and after washing twice, cell membrane was perforated by BD Phosflow™ Perm buffer III (558050, BD) and stained with PE-labeled anti-phosphorylated Smad2/3 antibody (562586, BD Phosflow™ Smad2 (pS465/pS467)/Smad3 (pS423/pS425), BD), a fluorescence-labeled antibody against B220 (BUV395, 563793, BD), and a fluorescence-labeled antibody against CD4 (PE-CF594, 562285, BD). Phosphorylated Smad2/3 in B cells were evaluated using a FACS analyzer, and the activity to input Smad signaling of the TGFβ3-modified antibody molecule was assessed.

As a result, when compared to no stimulation, mature TGFβ1 and mature TGFβ3 increased the proportion of phosphorylated Smad2/3; Plasmin-treated latent TGFβ3 and the TGFβ3-modified molecule increased the proportion of phosphorylated Smad2/3 similarly. On the other hand, 1D3-mF18 unconjugated with TGFβ3 or the plasmin protease alone did not affect the proportion of phosphorylated Smad2/3 compared to the no stimulation (Fig. 25). These results confirmed the concepts of B cell binding specificity and Smad signal input effect of the TGFβ3-modified antibody molecule.

### Suppressive effects on B cell proliferation

Spleen was isolated from 10-14-week-old male C57BL/6N mice purchased from Japan Charles River, and fractionated B cells (negative fraction) were purified using a Mouse B cell isolation kit (130-090-862, Miltenyi Biotec). Reactions were carried out using the CellTrace™ CFSE (Carboxyfluorescein diacetate succinimidyl ester) Cell Proliferation Kit for Flow Cytometry (C34554, Molecular Probes) for 20 minutes in a 37°C water bath, and then the cells were washed twice with PBS, and dispensed into a round-bottom 96-well plate at 10,000 cells/well. Then, latent TGFβ3, the TGFβ3-modified antibody molecule, and 0.5 µg/mL of the Plasmin protease were added. Furthermore, for stimulation of cell proliferation, 5 µg/mL of anti-IgM (715-005-140, Jackson), 5 µg/mL of anti-CD40 (HM40-3, BD Pharmingen), and 2 ng/mL of recombinant murine IL-4 (214-14, Peprotech) were added, and after culturing for three days at 37°C in a CO₂ incubator, the B cell proliferation-suppressive effect of the TGFβ3-modified antibody molecule was verified using a FACS analyzer (LSRFortessaX-20, BD) by examining cell division. A mature TGFβ1 recombinant protein (240-B-010/CF, R&D Systems) and a mature TGFβ3 recombinant protein (243-B3-010/CF, R&D Systems) were used as controls, and stimulation was started at the same time as antibody molecules.

While the B cell proliferation-suppressive effect of mature TGFβ1 was weak, the B cell proliferation-suppressive effect was confirmed for plasmin protease-treated TGFβ3-modified antibody molecule and also for latent TGFβ3, in a similar manner to mature TGFβ3 (Fig. 26). On the other hand, in 1D3-mF18 to which TGFβ3 is not conjugated, activity could not be confirmed at all, even with the plasmin protease treatment. This result indicates that the B cell proliferation- suppressive effects is far stronger in TGFβ3 than in TGFβ1, and that the biological activity can also be maintained by a TGFβ3-modified antibody molecule.

### Pharmacokinetic assessment of latent TGFβ3 and the TGFβ3-modified antibody molecule in mice

Because of the molecular properties, mature and latent TGFβ3 are predicted to show non-specific binding and a very rapid elimination rate in blood, and it is assumed that development as pharmaceutical agents in their original molecular form will face many problems. To sustainably suppress activated B cells, the molecular form of a TGFβ3-modified antibody molecule was examined to see whether blood half-life can be prolonged.

Latent TGF-β3 and the TGF-β3-modified antibody molecule were administered at 1 mg/kg to 6-week-old male C57BL/6J mice purchased from Japan Charles River through the caudal vein. After administration, blood was collected over time from the jugular vein, and plasma samples were obtained by subjecting the blood to centrifugation. The TGF-β3-modified antibody molecule and TGF-β3 concentrations in the plasma samples were quantified by ELISA. The concentration data of TGF-β3 and the TGF-β3-modified antibody molecule in plasma were analyzed, and the area under the plasma concentration curve (AUC_{inf}) and the half-life (T_{1/2}) were calculated.

The AUC and half-life of the antibody portion of the TGF-β3-modified antibody molecule were 8100 ng·day/mL and 2.09 day, respectively. On the other hand, the AUC_{inf} of TGF-β3 was 146 ng·hour/mL when administered as latent TGFβ3, but 982 ng·hour/mL when administered as a TGFβ3-modified antibody molecule. Furthermore, T_{1/2} of TGF-β3 was 0.116 hours when administered as latent TGFβ3, but 2.44 hours when administered as a TGF-β3-modified antibody molecule. This study revealed that administration of the TGF-β3-modified antibody molecule increases AUC and prolongs the T_{1/2} (Fig. 27 and Table 3).

These results suggest that modification to produce the molecular form of a TGFβ3-modified antibody molecule enables sustained prolongation of the Smad signal input effect as compared with latent TGFβ3, and suggests its possibility as a therapeutic agent.

**[Table 3]**

| Area under the plasma concentration curve (AUCinf) and plasma elimination half-life (T1/2) of TGFbeta3 after latent TGFbeta3 or TGF-beta3-modified antibody molecule was intravenously administered to mice at 1 mg/kg | | | |
|---|---|---|---|
| Test substance | | AUC_{inf} (ng*hour/mL) | T_{1/2} (hour) |
| Latent TGF-beta3 | mean | 146 | 0.116 |
| | SD | 22 | 0.016 |
| TGF-beta3-modified antibody molecule | mean | 982 | 2.44 |
| | SD | 66 | 0.36 |

| | | | |
|---|---|---|---|
| n=3 | | | |

### Assessment of the activity of the TGFβ3-modified antibody molecule in mice

The specific activity of the TGFβ3-modified antibody molecule to B cells will be evaluated by the following methods:
A) *Ex vivo*: The spleen and lymph nodes are isolated from B6.Cg-Tg(SBE/TK-luc)7Twc/J mice (SBE-Luc Tgm, 005999, Jackson Laboratory). They are reacted with the TGFβ3-modified antibody molecule and fluorescence-labeled antibodies against markers for T cells, B cells, NK cells, monocytes, dendritic cells, and such to confirm B cell specificity. Furthermore, the plasmin protease are added to the purified B cells to measure the luciferase activity. This way, intracellular signals induced by TGFβ3 will be evaluated.
B) *In vivo* imaging: An Alexa Fluor 647-labeled TGFβ3-modified antibody molecule is administered to SBE-Luc Tg mice, and *in vivo* imaging will be performed using IVIS Spectrum CT (Perkin Elmer). By using fluorescent signals to confirm that the distribution of antibodies and distribution from fluorescent signals of Smad signals mediated by TGFb3 overlap *in vivo,* the concept of the TGFβ3-modified antibody molecule, i.e., recruitment to B cells and the Smad signal input effect, will be confirmed.
C) Confirmation of Smad signals ins cells: Four proteins are prepared, which are 1D3-mF18, 1D3-mF18-latent TGFb3, and latent-TGFb3 shown in the above-mentioned Examples, as well as a modified antibody molecule that is linked with latent TGFb3 and having an antibody (different from the anti-CD19 antibody (1D3)) that does not bind to the B cell surface. These are administered to C57BL/6N mice or SBE-Luc Tg mice through the caudal vein, and the spleen and lymph nodes are isolated to compare and examine the proportions of pSmad. Furthermore, the four types of proteins mentioned above are administered through the caudal vein, and after isolating the spleen and lymph nodes, fluorescence-labeled antibodies against markers of T cells, B cells, NK cells, monocytes, dendritic cells, and such are used as indicators to isolate the respective cells and extract their RNAs. Then, by comprehensively analyzing the expressed genes using a next-generation sequencer, changes in expression of the TGFβ3 signal-related genes in the respective cells will be evaluated to confirm the concept of the TGFβ3-modified antibody molecule.

The above-mentioned evaluation prove that the TGFβ3-modified antibody molecule has an activity specific to the targeted B cells.

### Industrial Applicability

The activity of TGF-β3 to suppress B cell activation was newly found in the present invention, and the invention provides novel therapeutic strategies that use this activity for autoimmune diseases.

## Claims

1. A suppressor of B cell activation, which comprises TGF-β3.

2. The suppressor of claim 1, wherein the B cell is an autoreactive B cell.

3. The suppressor of claim 1 or 2, wherein the TGF-β3 suppresses antibody-production by B cells.

4. A therapeutic agent for an autoimmune disease, which comprises the suppressor of any one of claims 1 to 3.

5. A therapeutic agent for an autoimmune disease, which comprises a molecule in which TGF-β3 is linked with an antibody or an antibody fragment.

6. The therapeutic agent of claim 5, wherein the antibody or the antibody fragment comprises a variable region that recognizes a B cell.

7. The therapeutic agent of claim 6, wherein the variable region recognizes the B cell using any one or more of CD19, CD20, CD40, CD22, IL21R, BAFF-R, BCMA, TACI, CD27, and CD138 as a marker.

8. A therapeutic agent for an autoimmune disease, which comprises a multispecific antibody comprising a first variable region that recognizes TGF-β3 and a second variable region that recognizes a B cell.

9. The therapeutic agent of any one of claims 4 to 8, wherein the autoimmune disease is any one of systemic lupus erythematosus, pemphigus, multiple sclerosis, neuromyelitis optica, ANCA-associated vasculitis, rheumatoid arthritis, organ transplant rejection, Sjogren's syndrome, juvenile dermatomyositis, myasthenia gravis, or autoimmune thyroid disease including Graves' disease and Hashimoto's thyroiditis.

10. A medical kit comprising the therapeutic agent of any one of claims 4 to 9.


SEQUENCE LISTING

<110>  THE UNIVERSITY OF TOKYO
       CHUGAI SEIYAKU KABUSHIKI KAISHA

<120>  DEPRESSANT OF B CELL ACTIVITY AND THERAPEUTIC AGENT OF AUTOIMMUNE
        DISEASE

<130>  C1-X1410P

<150>  JP 2014-241193
<151>  2014-11-28

<160>  11

<170>  PatentIn version 3.5

<210>  1
<211>  412
<212>  PRT
<213>  Mus musculus

<400>  1

Met Lys Met His Leu Gln Arg Ala Leu Val Val Leu Ala Leu Leu Asn
1               5                   10                  15

Leu Ala Thr Ile Ser Leu Ser Leu Ser Thr Cys Thr Thr Leu Asp Phe
            20                  25                  30

Gly His Ile Lys Lys Lys Arg Val Glu Ala Ile Arg Gly Gln Ile Leu
        35                  40                  45

Ser Lys Leu Arg Leu Thr Ser Pro Pro Glu Pro Ser Val Met Thr His
    50                  55                  60

Val Pro Tyr Gln Val Leu Ala Leu Tyr Asn Ser Thr Arg Glu Leu Leu
65                  70                  75                  80

Glu Glu Met His Gly Glu Arg Glu Glu Gly Cys Thr Gln Glu Thr Ser
                85                  90                  95

Glu Ser Glu Tyr Tyr Ala Lys Glu Ile His Lys Phe Asp Met Ile Gln
            100                 105                 110

Gly Leu Ala Glu His Asn Glu Leu Ala Val Cys Pro Lys Gly Ile Thr
        115                 120                 125

Ser Lys Val Phe Arg Phe Asn Val Ser Ser Val Glu Lys Asn Gly Thr
    130                 135                 140

Asn Leu Phe Arg Ala Glu Phe Arg Val Leu Arg Val Pro Asn Pro Ser
145                 150                 155                 160

Ser Lys Arg Thr Glu Gln Arg Ile Glu Leu Phe Gln Ile Leu Arg Pro
                165                 170                 175

Asp Glu His Ile Ala Lys Gln Arg Tyr Ile Gly Gly Lys Asn Leu Pro
            180                 185                 190

Thr Arg Gly Thr Ala Glu Trp Leu Ser Phe Asp Val Thr Asp Thr Val
        195                 200                 205

Arg Glu Trp Leu Leu Arg Arg Glu Ser Asn Leu Gly Leu Glu Ile Ser
    210                 215                 220

Ile His Cys Pro Cys His Thr Phe Gln Pro Asn Gly Asp Ile Leu Glu
225                 230                 235                 240

Asn Val His Glu Val Met Glu Ile Lys Phe Lys Gly Val Asp Asn Glu
                245                 250                 255

Asp Asp His Gly Arg Gly Asp Leu Gly Arg Leu Lys Lys Gln Lys Asp
            260                 265                 270

His His Asn Pro His Leu Ile Leu Met Met Ile Pro Pro His Arg Leu
        275                 280                 285

Asp Ser Pro Gly Gln Gly Ser Gln Arg Lys Lys Arg Ala Leu Asp Thr
    290                 295                 300

Asn Tyr Cys Phe Arg Asn Leu Glu Glu Asn Cys Cys Val Arg Pro Leu
305                 310                 315                 320

Tyr Ile Asp Phe Arg Gln Asp Leu Gly Trp Lys Trp Val His Glu Pro
                325                 330                 335

Lys Gly Tyr Tyr Ala Asn Phe Cys Ser Gly Pro Cys Pro Tyr Leu Arg
            340                 345                 350

Ser Ala Asp Thr Thr His Ser Thr Val Leu Gly Leu Tyr Asn Thr Leu
        355                 360                 365

Asn Pro Glu Ala Ser Ala Ser Pro Cys Cys Val Pro Gln Asp Leu Glu
    370                 375                 380

Pro Leu Thr Ile Leu Tyr Tyr Val Gly Arg Thr Pro Lys Val Glu Gln
385                 390                 395                 400

Leu Ser Asn Met Val Val Lys Ser Cys Lys Cys Ser
                405                 410

<210>  2
<211>  431
<212>  PRT
<213>  Artificial Sequence

<220>
<223>  Mutant for Sortase linkage (signal sequence IL3 - Gly5- FLAG -
       mouse TGF-b3(C25S))

<400>  2

Met Val Leu Ala Ser Ser Thr Thr Ser Ile His Thr Met Leu Leu Leu
1               5                   10                  15

Leu Leu Met Leu Ala Gln Pro Ala Leu Ala Gly Gly Gly Gly Gly Asp
            20                  25                  30

Tyr Lys Asp Asp Asp Asp Lys Ser Leu Ser Leu Ser Thr Ser Thr Thr
        35                  40                  45

Leu Asp Phe Gly His Ile Lys Lys Lys Arg Val Glu Ala Ile Arg Gly
    50                  55                  60

Gln Ile Leu Ser Lys Leu Arg Leu Thr Ser Pro Pro Glu Pro Ser Val
65                  70                  75                  80

Met Thr His Val Pro Tyr Gln Val Leu Ala Leu Tyr Asn Ser Thr Arg
                85                  90                  95

Glu Leu Leu Glu Glu Met His Gly Glu Arg Glu Glu Gly Cys Thr Gln
            100                 105                 110

Glu Thr Ser Glu Ser Glu Tyr Tyr Ala Lys Glu Ile His Lys Phe Asp
        115                 120                 125

Met Ile Gln Gly Leu Ala Glu His Asn Glu Leu Ala Val Cys Pro Lys
    130                 135                 140

Gly Ile Thr Ser Lys Val Phe Arg Phe Asn Val Ser Ser Val Glu Lys
145                 150                 155                 160

Asn Gly Thr Asn Leu Phe Arg Ala Glu Phe Arg Val Leu Arg Val Pro
                165                 170                 175

Asn Pro Ser Ser Lys Arg Thr Glu Gln Arg Ile Glu Leu Phe Gln Ile
            180                 185                 190

Leu Arg Pro Asp Glu His Ile Ala Lys Gln Arg Tyr Ile Gly Gly Lys
        195                 200                 205

Asn Leu Pro Thr Arg Gly Thr Ala Glu Trp Leu Ser Phe Asp Val Thr
    210                 215                 220

Asp Thr Val Arg Glu Trp Leu Leu Arg Arg Glu Ser Asn Leu Gly Leu
225                 230                 235                 240

Glu Ile Ser Ile His Cys Pro Cys His Thr Phe Gln Pro Asn Gly Asp
                245                 250                 255

Ile Leu Glu Asn Val His Glu Val Met Glu Ile Lys Phe Lys Gly Val
            260                 265                 270

Asp Asn Glu Asp Asp His Gly Arg Gly Asp Leu Gly Arg Leu Lys Lys
        275                 280                 285

Gln Lys Asp His His Asn Pro His Leu Ile Leu Met Met Ile Pro Pro
    290                 295                 300

His Arg Leu Asp Ser Pro Gly Gln Gly Ser Gln Arg Lys Lys Arg Ala
305                 310                 315                 320

Leu Asp Thr Asn Tyr Cys Phe Arg Asn Leu Glu Glu Asn Cys Cys Val
                325                 330                 335

Arg Pro Leu Tyr Ile Asp Phe Arg Gln Asp Leu Gly Trp Lys Trp Val
            340                 345                 350

His Glu Pro Lys Gly Tyr Tyr Ala Asn Phe Cys Ser Gly Pro Cys Pro
        355                 360                 365

Tyr Leu Arg Ser Ala Asp Thr Thr His Ser Thr Val Leu Gly Leu Tyr
    370                 375                 380

Asn Thr Leu Asn Pro Glu Ala Ser Ala Ser Pro Cys Cys Val Pro Gln
385                 390                 395                 400

Asp Leu Glu Pro Leu Thr Ile Leu Tyr Tyr Val Gly Arg Thr Pro Lys
                405                 410                 415

Val Glu Gln Leu Ser Asn Met Val Val Lys Ser Cys Lys Cys Ser
            420                 425                 430

<210>  3
<211>  421
<212>  PRT
<213>  Artificial Sequence

<220>
<223>  Mutant for Latent TGF-b3 expression (signal sequence IgK - FLAG -
      mouse TGF-b3(C25S))

<400>  3

Met Glu Thr Asp Thr Leu Leu Leu Trp Val Leu Leu Leu Trp Val Pro
1               5                   10                  15

Gly Ser Thr Gly Asp Asp Tyr Lys Asp Asp Asp Asp Lys Ser Leu Ser
            20                  25                  30

Leu Ser Thr Ser Thr Thr Leu Asp Phe Gly His Ile Lys Lys Lys Arg
        35                  40                  45

Val Glu Ala Ile Arg Gly Gln Ile Leu Ser Lys Leu Arg Leu Thr Ser
    50                  55                  60

Pro Pro Glu Pro Ser Val Met Thr His Val Pro Tyr Gln Val Leu Ala
65                  70                  75                  80

Leu Tyr Asn Ser Thr Arg Glu Leu Leu Glu Glu Met His Gly Glu Arg
                85                  90                  95

Glu Glu Gly Cys Thr Gln Glu Thr Ser Glu Ser Glu Tyr Tyr Ala Lys
            100                 105                 110

Glu Ile His Lys Phe Asp Met Ile Gln Gly Leu Ala Glu His Asn Glu
        115                 120                 125

Leu Ala Val Cys Pro Lys Gly Ile Thr Ser Lys Val Phe Arg Phe Asn
    130                 135                 140

Val Ser Ser Val Glu Lys Asn Gly Thr Asn Leu Phe Arg Ala Glu Phe
145                 150                 155                 160

Arg Val Leu Arg Val Pro Asn Pro Ser Ser Lys Arg Thr Glu Gln Arg
                165                 170                 175

Ile Glu Leu Phe Gln Ile Leu Arg Pro Asp Glu His Ile Ala Lys Gln
            180                 185                 190

Arg Tyr Ile Gly Gly Lys Asn Leu Pro Thr Arg Gly Thr Ala Glu Trp
        195                 200                 205

Leu Ser Phe Asp Val Thr Asp Thr Val Arg Glu Trp Leu Leu Arg Arg
    210                 215                 220

Glu Ser Asn Leu Gly Leu Glu Ile Ser Ile His Cys Pro Cys His Thr

225                 230                 235                 240

Phe Gln Pro Asn Gly Asp Ile Leu Glu Asn Val His Glu Val Met Glu
                245                 250                 255

Ile Lys Phe Lys Gly Val Asp Asn Glu Asp Asp His Gly Arg Gly Asp
            260                 265                 270

Leu Gly Arg Leu Lys Lys Gln Lys Asp His His Asn Pro His Leu Ile
        275                 280                 285

Leu Met Met Ile Pro Pro His Arg Leu Asp Ser Pro Gly Gln Gly Ser
    290                 295                 300

Gln Arg Lys Lys Arg Ala Leu Asp Thr Asn Tyr Cys Phe Arg Asn Leu
305                 310                 315                 320

Glu Glu Asn Cys Cys Val Arg Pro Leu Tyr Ile Asp Phe Arg Gln Asp
                325                 330                 335

Leu Gly Trp Lys Trp Val His Glu Pro Lys Gly Tyr Tyr Ala Asn Phe
            340                 345                 350

Cys Ser Gly Pro Cys Pro Tyr Leu Arg Ser Ala Asp Thr Thr His Ser
        355                 360                 365

Thr Val Leu Gly Leu Tyr Asn Thr Leu Asn Pro Glu Ala Ser Ala Ser
    370                 375                 380

Pro Cys Cys Val Pro Gln Asp Leu Glu Pro Leu Thr Ile Leu Tyr Tyr
385                 390                 395                 400

Val Gly Arg Thr Pro Lys Val Glu Gln Leu Ser Asn Met Val Val Lys
                405                 410                 415

Ser Cys Lys Cys Ser
            420

<210>  4
<211>  412
<212>  PRT
<213>  Homo sapiens

<400>  4

<210>  5
<211>  431
<212>  PRT
<213>  Artificial Sequence

<220>
<223>  Mutant for Sortase linkage (signal sequence IL3 - Gly5- FLAG -
       human TGF-b3(C25S))

<400>  5

Met Val Leu Ala Ser Ser Thr Thr Ser Ile His Thr Met Leu Leu Leu
1               5                   10                  15

Leu Leu Met Leu Ala Gln Pro Ala Leu Ala Gly Gly Gly Gly Gly Asp
            20                  25                  30

Tyr Lys Asp Asp Asp Asp Lys Ser Leu Ser Leu Ser Thr Ser Thr Thr
        35                  40                  45

Leu Asp Phe Gly His Ile Lys Lys Lys Arg Val Glu Ala Ile Arg Gly
    50                  55                  60

Gln Ile Leu Ser Lys Leu Arg Leu Thr Ser Pro Pro Glu Pro Thr Val
65                  70                  75                  80

Met Thr His Val Pro Tyr Gln Val Leu Ala Leu Tyr Asn Ser Thr Arg
                85                  90                  95

Glu Leu Leu Glu Glu Met His Gly Glu Arg Glu Glu Gly Cys Thr Gln
            100                 105                 110

Glu Asn Thr Glu Ser Glu Tyr Tyr Ala Lys Glu Ile His Lys Phe Asp
        115                 120                 125

Met Ile Gln Gly Leu Ala Glu His Asn Glu Leu Ala Val Cys Pro Lys
    130                 135                 140

Gly Ile Thr Ser Lys Val Phe Arg Phe Asn Val Ser Ser Val Glu Lys
145                 150                 155                 160

Asn Arg Thr Asn Leu Phe Arg Ala Glu Phe Arg Val Leu Arg Val Pro
                165                 170                 175

Asn Pro Ser Ser Lys Arg Asn Glu Gln Arg Ile Glu Leu Phe Gln Ile
            180                 185                 190

Leu Arg Pro Asp Glu His Ile Ala Lys Gln Arg Tyr Ile Gly Gly Lys
        195                 200                 205

Asn Leu Pro Thr Arg Gly Thr Ala Glu Trp Leu Ser Phe Asp Val Thr
    210                 215                 220

Asp Thr Val Arg Glu Trp Leu Leu Arg Arg Glu Ser Asn Leu Gly Leu
225                 230                 235                 240

Glu Ile Ser Ile His Cys Pro Cys His Thr Phe Gln Pro Asn Gly Asp
                245                 250                 255

Ile Leu Glu Asn Ile His Glu Val Met Glu Ile Lys Phe Lys Gly Val
            260                 265                 270

Asp Asn Glu Asp Asp His Gly Arg Gly Asp Leu Gly Arg Leu Lys Lys
        275                 280                 285

Gln Lys Asp His His Asn Pro His Leu Ile Leu Met Met Ile Pro Pro
    290                 295                 300

His Arg Leu Asp Asn Pro Gly Gln Gly Gly Gln Arg Lys Lys Arg Ala
305                 310                 315                 320

Leu Asp Thr Asn Tyr Cys Phe Arg Asn Leu Glu Glu Asn Cys Cys Val
                325                 330                 335

Arg Pro Leu Tyr Ile Asp Phe Arg Gln Asp Leu Gly Trp Lys Trp Val
            340                 345                 350

His Glu Pro Lys Gly Tyr Tyr Ala Asn Phe Cys Ser Gly Pro Cys Pro
        355                 360                 365

Tyr Leu Arg Ser Ala Asp Thr Thr His Ser Thr Val Leu Gly Leu Tyr
    370                 375                 380

Asn Thr Leu Asn Pro Glu Ala Ser Ala Ser Pro Cys Cys Val Pro Gln
385                 390                 395                 400

Asp Leu Glu Pro Leu Thr Ile Leu Tyr Tyr Val Gly Arg Thr Pro Lys
                405                 410                 415

Val Glu Gln Leu Ser Asn Met Val Val Lys Ser Cys Lys Cys Ser
            420                 425                 430

<210>  6
<211>  421
<212>  PRT
<213>  Artificial Sequence

<220>
<223>  Mutant for Latent TGF-b3 expression (signal sequence IgK - FLAG -
       human TGF-b3(C25S))

<400>  6

Met Glu Thr Asp Thr Leu Leu Leu Trp Val Leu Leu Leu Trp Val Pro
1               5                   10                  15

Gly Ser Thr Gly Asp Asp Tyr Lys Asp Asp Asp Asp Lys Ser Leu Ser
            20                  25                  30

Leu Ser Thr Ser Thr Thr Leu Asp Phe Gly His Ile Lys Lys Lys Arg
        35                  40                  45

Val Glu Ala Ile Arg Gly Gln Ile Leu Ser Lys Leu Arg Leu Thr Ser
    50                  55                  60

Pro Pro Glu Pro Thr Val Met Thr His Val Pro Tyr Gln Val Leu Ala
65                  70                  75                  80

Leu Tyr Asn Ser Thr Arg Glu Leu Leu Glu Glu Met His Gly Glu Arg
                85                  90                  95

Glu Glu Gly Cys Thr Gln Glu Asn Thr Glu Ser Glu Tyr Tyr Ala Lys
            100                 105                 110

Glu Ile His Lys Phe Asp Met Ile Gln Gly Leu Ala Glu His Asn Glu
        115                 120                 125

Leu Ala Val Cys Pro Lys Gly Ile Thr Ser Lys Val Phe Arg Phe Asn
    130                 135                 140

Val Ser Ser Val Glu Lys Asn Arg Thr Asn Leu Phe Arg Ala Glu Phe
145                 150                 155                 160

Arg Val Leu Arg Val Pro Asn Pro Ser Ser Lys Arg Asn Glu Gln Arg
                165                 170                 175

Ile Glu Leu Phe Gln Ile Leu Arg Pro Asp Glu His Ile Ala Lys Gln
            180                 185                 190

Arg Tyr Ile Gly Gly Lys Asn Leu Pro Thr Arg Gly Thr Ala Glu Trp
        195                 200                 205

Leu Ser Phe Asp Val Thr Asp Thr Val Arg Glu Trp Leu Leu Arg Arg
    210                 215                 220

Glu Ser Asn Leu Gly Leu Glu Ile Ser Ile His Cys Pro Cys His Thr
225                 230                 235                 240

Phe Gln Pro Asn Gly Asp Ile Leu Glu Asn Ile His Glu Val Met Glu
                245                 250                 255

Ile Lys Phe Lys Gly Val Asp Asn Glu Asp Asp His Gly Arg Gly Asp
            260                 265                 270

Leu Gly Arg Leu Lys Lys Gln Lys Asp His His Asn Pro His Leu Ile
        275                 280                 285

Leu Met Met Ile Pro Pro His Arg Leu Asp Asn Pro Gly Gln Gly Gly
    290                 295                 300

Gln Arg Lys Lys Arg Ala Leu Asp Thr Asn Tyr Cys Phe Arg Asn Leu
305                 310                 315                 320

Glu Glu Asn Cys Cys Val Arg Pro Leu Tyr Ile Asp Phe Arg Gln Asp
                325                 330                 335

Leu Gly Trp Lys Trp Val His Glu Pro Lys Gly Tyr Tyr Ala Asn Phe
            340                 345                 350

Cys Ser Gly Pro Cys Pro Tyr Leu Arg Ser Ala Asp Thr Thr His Ser
        355                 360                 365

Thr Val Leu Gly Leu Tyr Asn Thr Leu Asn Pro Glu Ala Ser Ala Ser
    370                 375                 380

Pro Cys Cys Val Pro Gln Asp Leu Glu Pro Leu Thr Ile Leu Tyr Tyr
385                 390                 395                 400

Val Gly Arg Thr Pro Lys Val Glu Gln Leu Ser Asn Met Val Val Lys
                405                 410                 415

Ser Cys Lys Cys Ser
            420

<210>  7
<211>  25
<212>  DNA
<213>  Artificial Sequence

<220>
<223>  Egr2 promoter primer

<400>  7
agaccgcatt tactcttatc accag                                          25

<210>  8
<211>  25
<212>  DNA
<213>  Artificial Sequence

<220>
<223>  SV40 polyA specific primer

<400>  8
tgagtttgga caaaccacaa ctaga                                          25

<210>  9
<211>  414
<212>  PRT
<213>  Artificial Sequence

<220>
<223>  Heavy chain-V5-TB3

<400>  9

Ala Lys Thr Thr Pro Pro Ser Val Tyr Pro Leu Ala Pro Gly Ser Ala
1               5                   10                  15

Ala Gln Thr Asn Ser Met Val Thr Leu Gly Cys Leu Val Lys Gly Tyr
            20                  25                  30

Phe Pro Glu Pro Val Thr Val Thr Trp Asn Ser Gly Ser Leu Ser Ser
        35                  40                  45

Gly Val His Thr Phe Pro Ala Val Leu Gln Ser Asp Leu Tyr Thr Leu
    50                  55                  60

Ser Ser Ser Val Thr Val Pro Ser Ser Thr Trp Pro Ser Glu Thr Val
65                  70                  75                  80

Thr Cys Asn Val Ala His Pro Ala Ser Ser Thr Lys Val Asp Lys Lys
                85                  90                  95

Ile Val Pro Arg Asp Cys Gly Cys Lys Pro Cys Ile Cys Thr Val Lys
            100                 105                 110

Glu Val Ser Lys Val Phe Ile Phe Pro Pro Lys Pro Lys Asp Val Leu
        115                 120                 125

Thr Ile Thr Leu Thr Pro Lys Val Thr Cys Val Val Val Asp Ile Ser
    130                 135                 140

Lys Asp Asp Pro Glu Val Gln Phe Ser Trp Phe Val Asp Asp Val Glu
145                 150                 155                 160

Val His Thr Ala Gln Thr Gln Pro Arg Glu Glu Gln Phe Asn Ser Thr
                165                 170                 175

Phe Arg Ser Val Ser Glu Leu Pro Ile Met His Gln Asp Trp Leu Asn
            180                 185                 190

Gly Lys Glu Phe Lys Cys Arg Val Asn Ser Ala Ala Phe Pro Ala Pro
        195                 200                 205

Ile Glu Lys Thr Ile Ser Lys Thr Lys Gly Arg Pro Lys Ala Pro Gln
    210                 215                 220

Val Tyr Thr Ile Pro Pro Pro Lys Glu Gln Met Ala Lys Asp Lys Val
225                 230                 235                 240

Ser Leu Thr Cys Met Ile Thr Asp Phe Phe Pro Glu Asp Ile Thr Val
                245                 250                 255

Glu Trp Gln Trp Asn Gly Gln Pro Ala Glu Asn Tyr Lys Asn Thr Gln
            260                 265                 270

Pro Ile Met Arg Thr Asp Gly Ser Tyr Phe Val Tyr Ser Lys Leu Asn
        275                 280                 285

Val Gln Lys Ser Asn Trp Glu Ala Gly Asn Thr Phe Thr Cys Ser Val
    290                 295                 300

Leu His Glu Gly Leu His Asn His His Thr Glu Lys Ser Leu Ser His
305                 310                 315                 320

Ser Pro Gly Lys Gly Lys Pro Ile Pro Asn Pro Leu Leu Gly Leu Asp
                325                 330                 335

Ser Thr Arg Thr Gly Arg Gln Pro Arg Glu Glu Lys Lys Glu Cys Tyr
            340                 345                 350

Tyr Asn Leu Asn Asp Ala Ser Leu Cys Asp Asn Val Leu Ala Pro Asn
        355                 360                 365

Val Thr Lys Gln Glu Cys Cys Cys Thr Ser Gly Ala Gly Trp Gly Asp
    370                 375                 380

Asn Cys Glu Ile Phe Pro Cys Pro Val Gln Gly Thr Ala Glu Phe Thr
385                 390                 395                 400

Glu Met Cys Pro Arg Gly Lys Gly Leu Val Pro Ala Gly Glu
                405                 410

<210>  10
<211>  227
<212>  PRT
<213>  Artificial Sequence

<220>
<223>  nonVH-mF18Fc

<400>  10

Val Pro Arg Asp Ser Gly Cys Lys Pro Cys Ile Cys Thr Val Lys Glu
1               5                   10                  15

Val Ser Lys Val Phe Ile Phe Pro Pro Lys Pro Lys Asp Val Leu Thr
            20                  25                  30

Ile Thr Leu Thr Pro Lys Val Thr Cys Val Val Val Asp Ile Ser Lys
        35                  40                  45

Asp Asp Pro Glu Val Gln Phe Ser Trp Phe Val Asp Asp Val Glu Val
    50                  55                  60

His Thr Ala Gln Thr Gln Pro Arg Glu Glu Gln Phe Asn Ser Thr Phe
65                  70                  75                  80

Arg Ser Val Ser Glu Leu Pro Ile Met His Gln Asp Trp Leu Asn Gly
                85                  90                  95

Lys Glu Phe Lys Cys Arg Val Asn Ser Ala Ala Phe Pro Ala Pro Ile
            100                 105                 110

Glu Lys Thr Ile Ser Lys Thr Lys Gly Arg Pro Lys Ala Pro Gln Val
        115                 120                 125

Tyr Thr Ile Pro Pro Pro Lys Glu Gln Met Ala Lys Asp Lys Val Ser
    130                 135                 140

Leu Thr Cys Met Ile Thr Asp Phe Phe Pro Glu Asp Ile Thr Val Glu
145                 150                 155                 160

Trp Gln Trp Asn Gly Gln Pro Ala Glu Asn Tyr Lys Asn Thr Gln Pro
                165                 170                 175

Ile Met Asp Thr Asp Gly Ser Tyr Phe Val Tyr Ser Glu Leu Asn Val
            180                 185                 190

Gln Lys Ser Asn Trp Glu Ala Gly Asn Thr Phe Thr Cys Ser Val Leu
        195                 200                 205

His Glu Gly Leu His Asn His His Thr Glu Lys Ser Leu Ser His Ser
    210                 215                 220

Pro Gly Lys
225

<210>  11
<211>  387
<212>  PRT
<213>  Mus musculus

<400>  11

Thr Cys Thr Thr Leu Asp Phe Gly His Ile Lys Lys Lys Arg Val Glu
1               5                   10                  15

Ala Ile Arg Gly Gln Ile Leu Ser Lys Leu Arg Leu Thr Ser Pro Pro
            20                  25                  30

Glu Pro Ser Val Met Thr His Val Pro Tyr Gln Val Leu Ala Leu Tyr
        35                  40                  45

Asn Ser Thr Arg Glu Leu Leu Glu Glu Met His Gly Glu Arg Glu Glu
    50                  55                  60

Gly Cys Thr Gln Glu Thr Ser Glu Ser Glu Tyr Tyr Ala Lys Glu Ile
65                  70                  75                  80

His Lys Phe Asp Met Ile Gln Gly Leu Ala Glu His Asn Glu Leu Ala
                85                  90                  95

Val Cys Pro Lys Gly Ile Thr Ser Lys Val Phe Arg Phe Asn Val Ser
            100                 105                 110

Ser Val Glu Lys Asn Gly Thr Asn Leu Phe Arg Ala Glu Phe Arg Val
        115                 120                 125

Leu Arg Val Pro Asn Pro Ser Ser Lys Arg Thr Glu Gln Arg Ile Glu
    130                 135                 140

Leu Phe Gln Ile Leu Arg Pro Asp Glu His Ile Ala Lys Gln Arg Tyr
145                 150                 155                 160

Ile Gly Gly Lys Asn Leu Pro Thr Arg Gly Thr Ala Glu Trp Leu Ser
                165                 170                 175

Phe Asp Val Thr Asp Thr Val Arg Glu Trp Leu Leu Arg Arg Glu Ser
            180                 185                 190

Asn Leu Gly Leu Glu Ile Ser Ile His Cys Pro Cys His Thr Phe Gln
        195                 200                 205

Pro Asn Gly Asp Ile Leu Glu Asn Val His Glu Val Met Glu Ile Lys
    210                 215                 220

Phe Lys Gly Val Asp Asn Glu Asp Asp His Gly Arg Gly Asp Leu Gly
225                 230                 235                 240

Arg Leu Lys Lys Gln Lys Asp His His Asn Pro His Leu Ile Leu Met
                245                 250                 255

Met Ile Pro Pro His Arg Leu Asp Ser Pro Gly Gln Gly Ser Gln Arg
            260                 265                 270

Lys Lys Arg Ala Leu Asp Thr Asn Tyr Cys Phe Arg Asn Leu Glu Glu
        275                 280                 285

Asn Cys Cys Val Arg Pro Leu Tyr Ile Asp Phe Arg Gln Asp Leu Gly
    290                 295                 300

Trp Lys Trp Val His Glu Pro Lys Gly Tyr Tyr Ala Asn Phe Cys Ser
305                 310                 315                 320

Gly Pro Cys Pro Tyr Leu Arg Ser Ala Asp Thr Thr His Ser Thr Val
                325                 330                 335

Leu Gly Leu Tyr Asn Thr Leu Asn Pro Glu Ala Ser Ala Ser Pro Cys
            340                 345                 350

Cys Val Pro Gln Asp Leu Glu Pro Leu Thr Ile Leu Tyr Tyr Val Gly
        355                 360                 365

Arg Thr Pro Lys Val Glu Gln Leu Ser Asn Met Val Val Lys Ser Cys
    370                 375                 380

Lys Cys Ser
385